# EUROPEAN PATENT APPLICATION

(11) **EP 4 086 350 A1**
(43) Date of publication of application: **09.11.2022**
(21) Application number: 21172761.5
(22) Date of filing: 07.05.2021
(51) Int. Cl.: C12N 15/85

(54) **CONTROLLED GENE EXPRESSION METHODS AND MEANS**

(71) Applicant: IMBA-Institut für Molekulare Biotechnologie GmbH, 1030 Wien (AT)
(72) Inventor: The designation of the inventor has not yet been filed
(74) Representative: SONN Patentanwälte OG

(57) **Abstract**

The present invention provides a genetic element comprising: a splice donor site, a first recombinase recognition site, a splice branch point, a second recombinase recognition site, a splice acceptor site, wherein the splice branch point is at a distance of 10 to 56 nucleotides in length from the splice acceptor site, and its uses in controlled gene inactivation in a cell.

SEQUENCE LISTING

<110> Institut für molekulare Biotechnologie GmbH

<120> Short conditional intron

<130> r77577

<160> 22

<170> BiSSAP 1.3.6

<210> 1
<211> 201
<212> DNA
<213> Artificial Sequence

<220>
<223> intron insert

<400> 1
gtaagtaata acttcgtata gcatacatta tacgaagtta ttcaaggtta gaagacaggt 60

ttaaggagac caatagaaac tgggcttgtc gagacagaga agactcttgc gtttctgata 120

ggcacctatt ggtcttactg acatccactt tgccataact tcgtatagca tacattatac 180

gaagttattt tctctccaca g 201

<210> 2
<211> 189
<212> DNA
<213> Artificial Sequence

<220>
<223> intron insert

<400> 2
gtaagtaata acttcgtata aggtatccta tacgaagtta ttctctctgc ctattggggt 60

tacaagacag gtttaaggag accaatagaa actgggcatg tggagacaga gaagactctt 120

gggtttctga taggcactga cataacttcg tataaggtat cctatacgaa gttattttcc 180

ctccctcag 189

<210> 3
<211> 256
<212> DNA
<213> Artificial Sequence

<220>
<223> intron insert

<400> 3
taggcttgtc ccgtttccac agggctcttc taaggtaagt aataacttcg tataaggtat 60

cctatacgaa gttattctct ctgcctattg gggttacaag acaggtttaa ggagaccaat 120

agaaactggg catgtggaga cagagaagac tcttgggttt ctgataggca ctgacataac 180

ttcgtataag gtatcctata cgaagttatt ttccctccct caggacagaa gagcgggcaa 240

cttgccccat ccagtg 256

<210> 4
<211> 189
<212> DNA
<213> Artificial Sequence

<220>
<223> intron insert

<400> 4
aaaaaaaata acttcgtata aggtatccta tacgaagtta ttctctctgc ctattggggt 60

tacaagacag gtttaaggag accaatagaa actgggcatg tggagacaga gaagactctt 120

gggtttctga taggcactga cataacttcg tataaggtat cctatacgaa gttattttcc 180

ctccctcag 189

<210> 5
<211> 189
<212> DNA
<213> Artificial Sequence

<220>
<223> intron insert

<400> 5
gtaagtaata acttcgtata aggtatccta tacgaagtta taaaaaaaaa atattggggt 60

tacaagacag gtttaaggag accaatagaa actgggcatg tggagacaga gaagactctt 120

gggtttctga taggcactga cataacttcg tataaggtat cctatacgaa gttattttcc 180

ctccctcag 189

<210> 6
<211> 189
<212> DNA
<213> Artificial Sequence

<220>
<223> intron insert

<400> 6
gtaagtaata acttcgtata aggtatccta tacgaagtta ttctctctgc caaaaaaaaa 60

aacaagacag gtttaaggag accaatagaa actgggcatg tggagacaga gaagactctt 120

gggtttctga taggcactga cataacttcg tataaggtat cctatacgaa gttattttcc 180

ctccctcag 189

<210> 7
<211> 189
<212> DNA
<213> Artificial Sequence

<220>
<223> intron insert

<400> 7
gtaagtaata acttcgtata aggtatccta tacgaagtta ttctctctgc ctattggggt 60

taaaaaaaaa atttaaggag accaatagaa actgggcatg tggagacaga gaagactctt 120

gggtttctga taggcactga cataacttcg tataaggtat cctatacgaa gttattttcc 180

ctccctcag 189

<210> 8
<211> 189
<212> DNA
<213> Artificial Sequence

<220>
<223> intron insert

<400> 8
gtaagtaata acttcgtata aggtatccta tacgaagtta ttctctctgc ctattggggt 60

tacaagacag gaaaaaaaaa accaatagaa actgggcatg tggagacaga gaagactctt 120

gggtttctga taggcactga cataacttcg tataaggtat cctatacgaa gttattttcc 180

ctccctcag 189

<210> 9
<211> 189
<212> DNA
<213> Artificial Sequence

<220>
<223> intron insert

<400> 9
gtaagtaata acttcgtata aggtatccta tacgaagtta ttctctctgc ctattggggt 60

tacaagacag gtttaaggag aaaaaaaaaa actgggcatg tggagacaga gaagactctt 120

gggtttctga taggcactga cataacttcg tataaggtat cctatacgaa gttattttcc 180

ctccctcag 189

<210> 10
<211> 189
<212> DNA
<213> Artificial Sequence

<220>
<223> intron insert

<400> 10
gtaagtaata acttcgtata aggtatccta tacgaagtta ttctctctgc ctattggggt      60

tacaagacag gtttaaggag accaatagaa aaaaaaaaaa aggagacaga gaagactctt     120

gggtttctga taggcactga cataacttcg tataaggtat cctatacgaa gttattttcc     180

ctccctcag                                                             189


<210> 11
<211> 189
<212> DNA
<213> Artificial Sequence


<220>
<223> intron insert

<400> 11
gtaagtaata acttcgtata aggtatccta tacgaagtta ttctctctgc ctattggggt      60

tacaagacag gtttaaggag accaatagaa actgggcatg taaaaaaaaa aaagactctt     120

gggtttctga taggcactga cataacttcg tataaggtat cctatacgaa gttattttcc     180

ctccctcag                                                             189


<210> 12
<211> 189
<212> DNA
<213> Artificial Sequence


<220>
<223> intron insert

<400> 12
gtaagtaata acttcgtata aggtatccta tacgaagtta ttctctctgc ctattggggt      60

tacaagacag gtttaaggag accaatagaa actgggcatg tggagacaga gaaaaaaaaa     120

aggtttctga taggcactga cataacttcg tataaggtat cctatacgaa gttattttcc     180

ctccctcag                                                             189


<210> 13
<211> 189
<212> DNA
<213> Artificial Sequence


<220>
<223> intron insert

<400> 13

gtaagtaata acttcgtata aggtatccta tacgaagtta ttctctctgc ctattggggt 60

tacaagacag gtttaaggag accaatagaa actgggcatg tggagacaga gaagactctt 120

gaaaaaaaaa aaggcactga cataacttcg tataaggtat cctatacgaa gttattttcc 180

ctccctcag 189

<210> 14
<211> 189
<212> DNA
<213> Artificial Sequence

<220>
<223> intron insert

<400> 14
gtaagtaata acttcgtata aggtatccta tacgaagtta ttctctctgc ctattggggt 60

tacaagacag gtttaaggag accaatagaa actgggcatg tggagacaga gaagactctt 120

gggtttctga taaaaaaaaa aataacttcg tataaggtat cctatacgaa gttattttcc 180

ctccctcag 189

<210> 15
<211> 189
<212> DNA
<213> Artificial Sequence

<220>
<223> intron insert

<400> 15
gtaagtaata acttcgtata aggtatccta tacgaagtta ttctctctgc ctattggggt 60

tacaagacag gtttaaggag accaatagaa actgggcatg tggagacaga gaagactctt 120

gggtttctga taggcactga cataacttcg tataaggtat cctatacgaa gttataaaaa 180

aaaaatcag 189

<210> 16
<211> 189
<212> DNA
<213> Artificial Sequence

<220>
<223> intron insert

<400> 16
gtaagtaata acttcgtata aggtatccta tacgaagtta ttctctctgc ctattggggt 60

tacaagacag gtttaaggag accaatagaa actgggcatg tggagacaga gaagactctt 120

gggtttctga taggcactga cataacttcg tataaggtat cctatacgaa gttattttcc 180

ctcccaaaa                                                              189

<210> 17
<211> 34
<212> DNA
<213> Artificial Sequence

<220>
<223> recombinase recognition sequence

<400> 17
ataacttcgt ataaggtatc ctatacgaag ttat                                  34

<210> 18
<211> 34
<212> DNA
<213> Artificial Sequence

<220>
<223> recombinase recognition sequence

<400> 18
gaagttccta ttctctagaa agtataggaa cttc                                  34

<210> 19
<211> 34
<212> DNA
<213> Artificial Sequence

<220>
<223> recombinase recognition sequence

<400> 19
ataacttcgt atagcataca ttatacgaag ttat                                  34

<210> 20
<211> 21
<212> DNA
<213> Artificial Sequence

<220>
<223> sequencing track

<400> 20
tacgcaccat gcagaataca a                                               21

<210> 21
<211> 21
<212> DNA
<213> Artificial Sequence

<220>
<223> sequencing track

<400> 21
caccatgcag gtaagtaata a                                                21

<210> 22
<211> 21
<212> DNA
<213> Artificial Sequence

<220>
<223> sequencing track

<400> 22
cctccctcag aatacaaatg a                                                21

## Description

The present invention relates to the field of controlled gene activation and inactivation.

### Background of the invention

Despite the revolutionary advancement with CRISPR technology, the generation of conditional allele has not been as easy as knockout and knock-in alleles. To study essential genes such as housekeeping or developmentally required genes, conditional knockout (cKO) approach, allowing spatiotemporal control of gene knockout, is ideal as, otherwise, a simple knockout approach may cause early developmental lethality. For many years, recombinase systems, like the Cre/LoxP system, have been widely utilized to construct cKO alleles, by inserting two (LoxP) recombination sites in the adjacent introns of essential exon(s). The making of conditional alleles (so called 'floxed' alleles) in mice traditionally involved the use of mouse embryonic stem cells (mESCs) (Bouabe & Okkenhaug. Methods in Molecular Biology 315-336 (2013)). Recently, cKO alleles have also been generated via CRISPR/Cas9-mediated insertion of LoxP sites, which turned out to be rather challenging even with additional refinements (Guru-murthy et al. Genome Biology (2019) 20:171).

Conditional intron approaches have been attempted in the past as it allows a simple insertional mutagenesis with a fixed universal conditional intronic cassette (Economides et al. Proceedings of the National Academy of Sciences 110, E3179-E3188 (2013); Andersson-Rolf et al. Nature Methods 14, 287-289 (2017); WO 2017/203275 A1; Guzzardo et al. Scientific Reports 7, (2017); WO 2018/096356 A1), but it has not been well utilized in animal models due to their long length (Economides et al. and Andersson-Rolf et al.) or an unexpected hypomorphic effect, a lowered gene expression of the targeted gene even before triggering the insert (Guzzardo et al.).

It is a goal of the invention to provide a conditional allele with reduced or no hypomorphic effect and that is easy to utilize in various organisms.

### Summary of the invention

The present invention provides a genetic element comprising: a splice donor site, a first recombinase recognition site, a splice branch point, a second recombinase recognition site, a splice acceptor site, wherein the splice branch point is at a distance of 10 to 56 nucleotides in length from the splice acceptor site.

The invention further provides a genetic vector comprising the genetic element.

Further provided is a method of providing a cell with a conditionally de-activatable gene, comprising providing a cell, introducing the genetic element into an exon of a gene in a cell, or introducing a gene with the genetic element into a cell.

Further provided is a cell comprising a gene with two or more exons and at least one intron, wherein the intron comprises the genetic element.

Also provided is a non-human animal comprising one or more cells of the invention.

Further provided is a method of inactivating expression of a functional gene in a cell or non-human animal, comprising providing a cell or a non-human animal of the invention and activating recombination at the recombinase recognition sites in the cell or in a cell in the non-human animal.

Further provided is a method of investigating the function of a gene, comprising inactivating a functional gene according to the method of the invention and comparing the inactivated gene's effect in the cell or non-human animal to a cell or non-human animal without inactivation of the gene or to a cell or non-human animal without the genetic element.

Also provided is a kit suitable for integrating an intron into a target gene comprising a genetic element of the invention and a Cas encoding nucleic acid.

All the above aspects of the invention are related and the following detailed description of particular embodiments relate to all aspects alike, even when descripted with focus on one embodiment. E.g. a description of the genetic element also relates to the method of incorporating it into a cell or animal or using it and the cells, animals or kits comprising the element. Descriptions of methods relate to the genetic element in terms of suitabilities of the genetic element for such methods. Descriptions of methods also relate to products of the methods as obtainable by the methods. The kit may comprise any part or component as used in the methods and may be suitable to perform the methods. The kit may of course also be used in the methods.

### Detailed description of the invention

The present invention provides a conditional intron system that is suitable for conditional knock-out approaches. The intron can be incorporated into an allele and activated at a time of choice in the living system during any time of biological development.

The generation of conditional allele using CRISPR technology is still challenging. The invention provides a Short Conditional intrON (also termed "SCON") that enables a rapid generation of conditional allele. For example, it can be used with a simple one-step zygote injection, among other uses. SCON has conditional intronic function in various organisms, including vertebrate species, and its target insertion is as simple as CRISPR/Cas9-mediated gene tagging.

In particular, the invention provides a genetic element comprising: a splice donor site, a first recombinase recognition site, a splice branch point, a second recombinase recognition site, a splice acceptor site, wherein the splice branch point is at a distance of 10 to 56 nucleotides in length from the splice acceptor site. The term "distance" as used herein refers to a number of nucleotides in length between the sites, not counting the nucleotides of the sites themselves. E.g. the nucleotides after "CTGAC", as an example of a branch point, and up to but excluding "AG", as an example of a splice acceptor, site can be counted. In preferred embodiments, the distance between the splice branch point and the splice acceptor site is 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 52, 53, 54 or 55 nucleotides. A preferred distance range is 35 to 50 nucleotides, e.g. 40 to 48 nucleotides, in length between the splice branch point and the splice acceptor site.

The genetic element of the invention can also be referred to as "nucleic acid construct" or "artificial intron" or "SCON".

The genetic element can be a nucleic acid molecule, preferably DNA. It may also be RNA. RNA genetic elements are preferably reverse transcribed into DNA before or during use. In case of RNA genetic elements, all disclosures of DNA sequences herein in preferred elements read on the corresponding RNA sequence, in particular with T being U. The nucleotides A, G, C T/U refer to the function in the genetic code. Chemically, the nucleotides can be A, G, C T/U nucleotide molecules or nucleotide molecule moieties (in a polynucleotide molecule), respectively, or any other nucleotides corresponding to the same code, such as modified nucleotides, like pseudouridine.

Any sequence herein may also be provided in the complementary reverse sequence, i.e. the anti-sense strand to the sequence provided herein. The genetic element as for use can be provided by generating the sense strand to such an anti-sense strand.

The genetic element may be provided as single strand or double strand nucleic acid, double strands are preferred for increased stability.

It is also beneficial when the splice branch point is close to one of the recombinase recognitions sites, in particular the second recombinase recognition site. In preferred embodiments, the splice branch point is at a distance of 0 to 11 nucleotides in length, e.g. at a distance of 1, 2, 3, 4, 5, 6, 7, 8, 9 or 10 nucleotides in length, from the second recombinase recognition site. The splice branch point may be directly adjacent to the second recombinase recognition site. In cases where there are more than one splice branch points, e.g. two or more splice branch points, the splice branch point closer to the second recombinase recognition site is considered.

In further preferred embodiments the splice donor site is at a distance of 80 to 5000 nucleotides in length, preferably 100 to 4500 nucleotides in length, from the splice acceptor site. Particular preferred nucleotides in length (nt) between the splice donor site and the splice acceptor site are 110 to 4000 nt, preferably 120 to 3500 nt, 130 to 3000 nt, 140 to 2500 nt, 150 to 2000 nt, 160 to 1500 nt, nucleotides in length. Further ranges are up to 1000 nt, up to 800 nt, up to 500 nt, up to 400 nt or up to 300 nt or up to 190 nt. Upper range limits can be combined with any of the lower range limits, such as 110 to 400 nt, preferably 120 to 350 nt, 130 to 300 nt, 140 to 250 nt, 150 to 200 nt, 160 to 190 nt, nucleotides in length. A shorter distance, such as below 500 nt or below 400 nt have the advantage of reduced costs and time efforts when handling the genetic element.

Preferably, a sequence of 10 nucleotides directly 5' adjacent to the splice acceptor site contains at least 8, preferably at least 9 or 10, pyrimidine nucleotides, preferably of which at least 3 nucleotides are C and/or preferably at least 3 nucleotides are T. This sequence may also be referred to as a "polypyrimidine tract". Substantial purine base (A, G, but especially A) content in this sequence may lead to an unwanted hypomorphic effect. Preferably the at least 8 pyrimidine nucleotides are directly 5' adjacent to the splice acceptor site.

In particular preferred embodiments of all aspects of the invention, the splice donor site comprises the nucleic acid sequence GTPuAG, with Pu being a purine base e.g. A or G, such as in GTAAG and GTGAG. A T nucleotide may follow the sequence GTPuAG in preferred embodiments; accordingly, the genetic element may comprise the sequence GTPuAGT, such as GTAAGT or GTGAGT at a splice donor site. The GT nucleotides in the sequence GTPuAG are most significant. Preferably the splice donor site contains the nucleotides GT. In preferred embodiments of the invention all distances given above for distances between the splice donor site and a further site may also be given as distance from the GT nucleotides of the splice donor site to that further site, plus 3 nucleotides (accounting for the PuAG sequence). The splice donor site may be preceded by the sequence MAG with M being C or A. Further splice donor sites are reported in Ohshima et al., J. Mol. Biol. 195, 247-259, 1987.

In preferred embodiments, the splice branch point comprises the nucleic acid sequence CTPuAPy, wherein Pu refers to a purine base, such as G or A and Py refers to a pyrimidine base, such as C and T or U. The splice branch point may comprise the sequences CTGAT, CTGAC, CTAAT or CTAAC. The A between Pu and Py is most significant. Preferably the splice branch point comprises an A, preferably between a Pu and a Py. In preferred embodiments of the invention all distances given above for distances between the splice branch point and a further site may also be given as distance from the A nucleotide of the splice branch point to that further site, plus 1 nucleotide for following further sites in 3' direction (accounting for the Py nucleotide); or plus 3 nucleotides for preceding further sites in 5' direction (accounting for the CTPu sequence). For example, the distance between the A of the splice branch point and the splice acceptor site can be 11 to 57 nucleotides in length, such as 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 52, 53, 54, 55 or 56 nucleotides. A preferred distance range is 36 to 51 nucleotides, e.g. 41 to 49 nucleotides, in length between the A of the splice branch point and the splice acceptor site. Further splice branch points are disclosed in Zhuang et al., PNAS 86, 2752-2756, 1989.

In preferred embodiments, the splice acceptor site comprises the sequence AG. In some embodiments, there may be a pyrimidine nucleotide ("Py"), in particular C or T (or U in case of RNA), directly preceding the splice acceptor site, preferably AG, such as in the sequence PyAG, in particular CAG or TAG. The AG nucleotides in the splice acceptor site are very efficient for splicing and are preferably contained in the splice acceptor site. Preferably the splice acceptor site comprises an A nucleotide. In preferred embodiments of the invention all distances given above for distances between the splice acceptor site and a further site may also be given as distance from the A nucleotide of the splice acceptor site to that further site. Further splice acceptor sites are disclosed in Seif et al., Nucleic Acids Res. 6(10), 3387-98, 1979.

The expression "recombinase recognition site" is also referred to as just "recombinase site" or recombinase target site or recombinase recognition targets. Preferably the first and second recombinase recognition sites are selected from a tyrosine recombinase site (recombinase sites of tyrosine-type site-specific recombinases (T-SSRs)). Example recombinases and sites are Flp (flippase), which binds to flippase recognition target (frt) sites. Preferred sites are lox (Cre) and frt (Flp). A preferred lox site is loxP that is derived from a bacteriophage P1 sequence. Other lox sites are e.g. Lox 511, Lox 5171, Lox 2272, M2, M3, M7, M11, lox 66 or lox 71. Further preferred recombinase recognition sites besides the already mentioned Cre/Lox and Flp/Frt sites are Dre/Rox, Vika/vox, VCre/VloxP, SCre/SloxP, λ-Int/attP, R/RRT, Kw/KwRT, Kd/KdRT, B2/B2RT, B3/B3RT (Meinke et al., Chem. Rev. 2016, 116, 20, 12785-12820, incorporated herein by reference). Dre/Rox and Vika/vox are particularly useful in mammalian cells or systems.

Especially preferred are a loxP or FRT site, more preferred a loxP site, even more preferred a loxP site comprising the nucleic acid sequence ATAACTTCGTATAAGGTATCCTATACGAAGTTAT (SEQ ID NO: 17); a lox 66 site or a lox 71 site, a FRP site, a FRT site, especially preferred a FRT site comprising the nucleic acid sequence GAAGTTCCTATTCTCTAGAAAGTATAGGAACTTC (SEQ ID NO: 18).

SEQ ID NO: 17 is a Lox2372 site. Another preferred loxP site is LoxP-wt (ATAACTTCGTATAGCATACATTATACGAAGTTAT, SEQ ID NO: 19). FRT sites can be selected from FRT-wt, F1-FRT, F3-FRT and others. Of these, FRT-wt worked best and had no discernible hypomorphic effect. FRT-wt is particularly preferred.

In some embodiments, the genetic element comprises two or more splice branch points, preferably wherein two splice branch points are at a distance of 1 to 10 nucleotides in length to each other. In other embodiments, the genetic element comprises only one branch point.

All of these embodiments can of course be combined, e.g. as shown in the examples. For example, the inventive genetic element comprises the sequences, in 5' to 3' direction: a splice donor site, a first recombinase recognition site, a splice branch point comprising an A nucleotide, preferably in the sequence PuAPy, a second recombinase recognition site, a splice acceptor site comprising an A nucleotide, preferably in the sequence AG, wherein the A of the splice branch point is at a distance of 11 to 57 nucleotides in length from the A of the splice acceptor site. Distances are nucleotide between these A's not counting the A's themselves. Said distance may be 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 52, 53, 54, 55 or 56 nucleotides in length. Pu are purine bases and Py are pyrimidine bases as mentioned above. In a preferred combination, the inventive genetic element comprises the sequences, in 5' to 3' direction: a splice donor site comprising the sequence GTPuAG, a first recombinase recognition site, a splice branch point comprising the sequence CTPu**A**Py, a second recombinase recognition site, a splice acceptor site comprising the sequence **A**G, wherein the A in bold of the splice branch point is at a distance of 11 to 57 nucleotides in length from the A in bold of the splice acceptor site. Distances are nucleotide between these A's not counting the A's themselves. Said distance may be as given above, i.e. any number from 11 to 57 in preferred embodiments. Pu are purine bases and Py are pyrimidine bases as mentioned above.

Preferred examples of the genetic element of the invention are provided in SEQ ID NOs: 2, 3, 5, 6, 7, 8, 9, 10, 11, 12, 13. The invention also provides a genetic element comprising a sequence of any one of SEQ ID NOs: 2, 3, 5, 6, 7, 8, 9, 10, 11, 12, 13 or a sequence with at least 50%, preferably least 60%, least 70%, least 80%, least 90%, least 95%, least 98%, sequence identity to any one of SEQ ID NOs: 2, 3, 5, 6, 7, 8, 9, 10, 11, 12, 13. SEQ ID NO: 2 and any sequence variant with the given identities are particularly preferred among these sequences.

The invention further relates to a genetic vector comprising the genetic element of the invention. The genetic vector can be an expression or integration vector, a single strand DNA oligo template, a double strand DNA template, a transposon, or a viral vector, preferably an adenoviral, adeno-associated viral, retro- or lentiviral vector, comprising the genetic element of the invention. The vector preferably comprises DNA or RNA. The vector may be used to transfect or transform a cell. The genetic element may be integrated into the genome of the cell.

The genetic element can be introduced as an intron into a gene of a cell and/or it is possible to introduce a gene or a genetic coding element with a genetic element of the invention as an intron, to a cell. Accordingly, the invention further provides a method of providing a cell with a conditionally deacti-vatable gene, comprising providing a cell, introducing the genetic element of the invention into an exon of a gene in a cell, or introducing a gene with the genetic element of the invention into a cell. Such a genetic element can be introduced using a vector of the invention. The genetic element can be inserted into an exon of the gene or it can be used to replace an intron in a gene with the inventive genetic element. It is also possible to modify an endogenous intron of a gene to resemble a genetic element of the invention so that the result is a genetic element of the invention in the intron. In essence, a gene can be created with the genetic element of the invention flanked by exon sequences. These are all options for introducing an intron into a gene. In preferred embodiments, genetic element of the invention is inserted into a sequence containing a splice junction consensus sequence selected (A/C)-A-G-(A/G) (Burset et al. Nucleic Acids Res. 28, 4364-4375 (2000)) or (A/G/C)-(A/T/G)-G-(A/T/G/C) (Ma et al. PLoS One 10, 1-12 (2015)), wherein nucleotides in brackets are alternatives at its position.

This allows the controlled expression of a gene that comprises the genetic element of the invention as an intron. The controlled gene expression of the invention is possible by introducing the genetic element of the invention into an exon which is still allowing an unaltered gene expression of the target gene since the genetic element of the invention is spliced. Following recombinase mediated intron function inactivation at a desired point in time the gene becomes inoperative due to lack of intron splicing. E.g. the non-spliced intron leads to transcription or translation stop or to the expression of a non-functional gene.

The inventive method may comprise introducing into a cell a sequence-specific nuclease that cleaves a sequence within an exon of the target gene thereby introducing the genetic element of the invention into the target gene. Preferably the introduction into an exon of a gene comprises CRISPR-Cas, preferably CRISPR-Cas9, mediated insertion. Further methods for the introduction are with a Zinc finger nuclease, TALEN, Cpf1, and other Cas9-related systems. The genetic element can be introduced by homology directed repair (HDR) or non-homologous end joining (NHEJ) .

CRISPR gene editing has facilitated the investigation of gene functions by precise gene knockouts or knock-ins. Upon gRNA-directed double-strand breaks (DSBs), the preferred repair pathway, non-homologous end-joining (NHEJ), often leads to random insertions or deletions (INDELS), where out-of-frame mutations can lead to partial or complete gene loss of function. Using a DNA template, DSBs can also be repaired via homology directed repair (HDR), which allows precise knock-ins of various sequences to target loci. Due to the versatility of CRISPR/Cas9 system and wide applicability, it has been utilized in numerous cell lines and lab organisms spanning the entire biological and biomedical research fields (Pickar-Oliver, A. & Gersbach, C., Nature Reviews Molecular Cell Biology 20, 490-507 (2019)). Accordingly, CRISPR gene editing can be used to introduce the genetic element of the invention into a target gene.

The invention further provides a cell comprising a gene with two or more exons and at least one intron, wherein the intron comprises the genetic element of the invention. The intron may be located between two exons. The cell may be obtainable by the inventive method. In preferred embodiments the cell is a eukaryotic cell, in particular preferred a vertebrate cell, such as a mammalian cell, a fish cell, a bird cell, an amphibian cell, a marsupial cell, a reptile cell; or an invertebrate, such as arthropod, a mollusk, an annelid or a cnidarian. Also possible are insect cells, plant cells, bacterial cells, fungi cells etc. Preferably the cells are selected from a differentiated cell or a pluripotent cell. In some embodiments, human totipotent cells are excluded. In other embodiments, any totipotent cell is excluded. The cell may be from a cell line, a cell culture cell or an isolated cell originating from but removed from a non-human animal or a human. The genetic element of the invention can be used as intron in a gene in pluripotent stem cells, cell lines, organoids, primary cells; such as human pluripotent stem cells, human cell lines, human organoids, primary human cells; or from any of the above organisms.

Conditional gene inactivation with the genetic element of the invention can be used to reduce viability or survivability of cells, such as by placing it in essential genes. Conditional gene inactivation can be used in removing transplanted human cells when, e.g. they are no longer needed in a cell therapy. For example, one can use the genetic element in CAR-T cells so that all treated CAR-T cells can be killed when the CAR-T cells are no longer needed or become detrimental for a recipient.

Further provided is a non-human animal comprising one or more cells of the invention. The non-human animal may be any organism mentioned above, e.g. a vertebrate, such as a mammal, a fish, a bird, an amphibian, a marsupial, a reptile. The cells of the invention may be provided or introduced to a human or a non-human animal.

The present invention further relates to a method of inactivating expression of a functional gene in a cell or non-human animal, comprising providing a cell of the invention or a non-human animal of the invention and activating recombination at the recombinase recognition sites in the cell of the invention or in a cell in the non-human animal of the invention. Activation of the recombinase recognition sites leads to an excision of the sequence between the recombinase recognition sites and thereby removal of the splice branch point, thereby removing the potential to splice the genetic element. For this, in all embodiments of the invention, the recombinase recognition sites should be in the same orientation. By removing the splicing potential, the gene containing the inventive genetic element will no longer be expressed properly as the genetic element without splicing leads to a malfunctioning gene, e.g. by containing a non-functional or disruptive sequence to the expressed gene, or an abrogated expression, such as by a premature termination. For a premature termination, the inventive genetic element preferably contains one or more stop codons. Preferably there is at least one stop codon in each reading frame, e.g. as described in WO 2018/096356 A1.

The method may comprise the step of introducing or activating a recombinase in the cell thereby excising or disrupting the branch point and abrogating splicing of the genetic element of the invention with the SCON. The recombinase acts on the first and second recombinase sites and excises sequence in between. Activation or introduction may comprise initiating expression of the recombinase or providing an activator for it. For example, recombinases can be expressed with a receptor that mediates activation. An example thereof is CreER, a Cre recombinase with an estrogen receptor (ER) that leads to the activation of the Cre part upon ER ligand binding, such as binding of a ligand like tamoxifen or 4-hydroxytamoxifen. A preferred type of CreER is CreER^{t2}.

The inventive genetic element can be used for targeting an endogenous gene in a cell. Alternatively, the overexpression of the inventive genetic element can be used to investigate the activity of a promoter, a transcription factor, a transcription cofactor, splicing factors, and regulation of splicing. For example, it is possible to study a reduced expression, e.g. a knock-out, or overexpression of such factors to screen for depleted or elevated expression, compared without these factors. For such studies, the genetic element of the invention may be in an intron of a reporter gene such as a fluorescent protein, like GFP, or an antibiotic resistance gene.

The invention provides a method of investigating the function of a gene, comprising inactivating a functional gene according to the method of the invention and comparing the inactivated gene's effect in the cell or non-human animal to a cell or non-human animal without inactivation of the gene or to a cell or non-human animal without the genetic element of the invention. The comparison to a state without the genetic element of the invention or to the state without inactivation serves as a control to identify differences caused by the gene under investigation that comprises the genetic element of the invention. The inactivation of a functional gene may lead to an observable effect, such as altered cell function, viability of gene expression. As mentioned above, the functional gene may be a reporter gene with an expected expression pattern difference before and after inactivation, wherein other activities of the cell affecting the reporter gene, such as splicing or promoter functions, are investigated.

Further provided is a kit suitable for integrating an intron into a target gene comprising a genetic element of the invention and a Cas encoding nucleic acid and/or a Cas protein, preferably further comprising a CRISPR-Cas guide nucleic acid targeting an exon in the target gene. The Cas protein is preferably Cas9; the Cas encoding nucleic acid is preferably a Cas9 encoding nucleic acid. The Cas enzyme may be Cas1, Cas2, Cas3, Cas9, dCas9, Cas10 or Cas12a. The CRISPR/Cas method typically comprises the use of single guide RNA (sgRNA) that comprises both the crRNA (CRISPR RNA) and tracrRNA (trans-activating crRNA) as a single construct. The crRNA is also referred to as guideRNA for containing the DNA guiding sequence. The guideRNA may be specific for a gene wherein the inventive genetic element may be inserted into. The tracrRNA and the crRNA can be linked to form a single molecule, i.e. the single guide RNA (sgRNA). tracrRNA and crRNA hybridize in a complementary region. This complementary region can be used for the linkage and may form, together with a linkage, a stem-loop, called the crRNA:tracrRNA stem loop herein. Since this region in most cases mediates binding to a Cas protein, it may also be referred to as Cas binding element. Site-specific cleavage occurs at locations determined by both base-pairing complementarity between the crRNA and the target protospacer DNA, and a short motif [referred to as the protospacer adjacent motif (PAM)] juxtaposed to the complementary region in the target DNA. The target DNA may be in any DNA molecule that should be modified. It may be of a gene that shall be modified. A typical use of the CRISPR/Cas system is to introduce mutations or modifications that allows the insertion of the genetic element of the invention. The design of sgRNAs is by now conventional, as reviewed e.g. by Ciu et al. (Interdisciplinary Sciences Computational Life Sciences 2018, DOI: 10.1007/s12539-018-0298-z) or Hwang et al. (BMC Bioinformatics 19, 2018:542). Many tools exist that can be used according to the invention to generate a sgRNA sequence targeting a gene of interest.

The kit may comprise a guideRNA targeting a gene of interest. The kit may also comprise a tracrRNA suitable for a CRISPR-Cas method. The guideRNA and tracrRNA may be in a single RNA molecule, as in a sgRNA. Alternatively or in addition, the kit may comprise a nucleic acid encoding any of these RNA (any of guideRNA, tracrRNA and/or sgRNA).

The kit can be used for various uses, such for zygote pronu-clear injection, zygote cytoplasmic transfection, or for 2-cell cytoplasmic transfection.

The invention further provides a method of introducing an intron sequence into an exon or between two exons of a gene, comprising the steps of selecting the exon or one of the two exons, respectively, which is positioned within the first 50% base pairs (bp) of a protein coding-sequence of the gene; and the intron is inserted into an intron insertion site containing either a stringent splice junction consensus sequence or a flexible splice junction consensus sequence; and wherein after the introduction of the intron the intron is separating exons on the intron's 5' and 3' sides with the exons being each at least 60 bp in length.

In preferred embodiments, the stringent splice junction consensus sequence is A/C)-A-G-(A/G) (Burset et al. Nucleic Acids Res. 28, 4364-4375 (2000). In further preferred embodiments, the flexible splice junction consensus sequence comprised the sequence (A/G/C)-(A/T/G)-G-(A/T/G/C) (Ma et al. PLoS One 10, 1-12 (2015)).

Preferably, an exon that is to be spit by introducing the intron into the exon is at least and the exon is at least 120 bp in length.

The protein coding-sequence of the gene can be of a genome, e.g. be located on a chromosome. The gene can be of a cell or organism as described above for the genetic element of the invention (SCON), such as in a cell from a cell line, a cell culture cell or an isolated cell originating from but removed from a non-human ani-mal or a human.

The intron can be used to replace an existing intron, e.g. as embodiment of introducing the intron sequence between two exons of a gene, or introduced into an exon that in the target gene would not have an intron at the selected site of intron insertion.

The intron may be a conditional intron that can have an intron function during splicing under selectable or controllable conditions, i.e. the intron can be inactivated or activated. For such embodiments, the functional intron may have a splice donor site, a splice branch point, and a splice acceptor site, functionally positioned to act as an intron during splicing, e.g.. being excised from a transcript. The functional position may be disrupted by recombinase recognition sites, that may change the functional positioning by action of a recombinase. A preferred intron is a genetic element of the invention (SCON) as described above. The SCON is an example of an intron with splicing function that can be deactivated upon recombinase action, turning the intron non-functional. E.g. the intron may comprise a splice donor site, a first recombinase recognition site, a splice branch point, a second recombinase recognition site, a splice acceptor site. A pair of recombinase recognition sites, e.g. a first and a second recombinase recognition sites, may be used to alter the availability of a site necessary for splice function, such as the splice donor site, a splice branch point, and/or a splice acceptor site, for splicing. The alteration may be a functional removal or functional activation of splicing function. A functional removal may be a physical removal of one or more of the sites, such as an excision, by the recombinase. Another functional alteration may turn an inactive site active, such as by removing inhibitory RNA structures by the recombinase. As mentioned above, a recombinase may remove the part or site flanked by a pair of recombinase recognition sites.

Throughout the present disclosure, the articles "a", "an", and "the" are used herein to refer to one or to more than one (i.e., to at least one) of the grammatical object of the article.

As used herein, words of approximation such as, without limitation, "about", "substantial" or "substantially" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by e.g. ±10%.

As used herein, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The "comprising" expressions when used on an element in combination with a numerical range of a certain value of that element means that the element is limited to that range while "comprising" still relates to the optional presence of other elements. E.g. the element with a range may be subject to an implicit proviso excluding the presence of that element in an amount outside of that range. As used herein, the phrase "consisting essentially of" requires the specified integer(s) or steps as well as those that do not materially affect the character or function of the claimed invention. As used herein, the closed term "consisting" is used to indicate the presence of the recited elements only.

The present invention is further described by the following figures and examples, without necessarily being limited to these embodiments of the invention.

### Figures

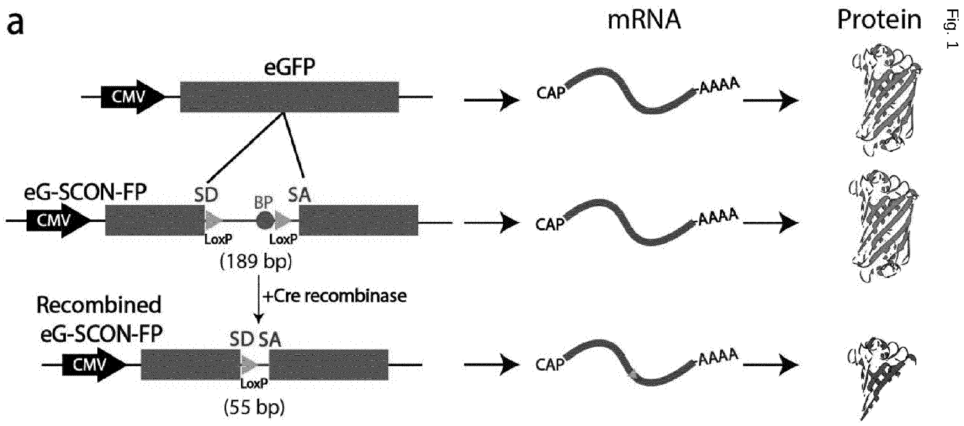

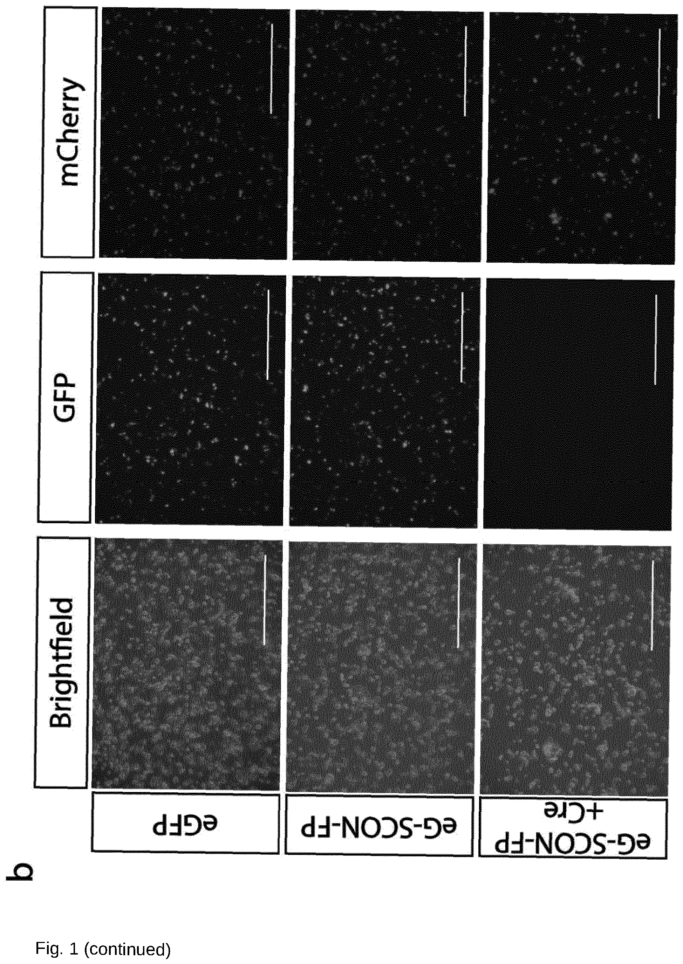

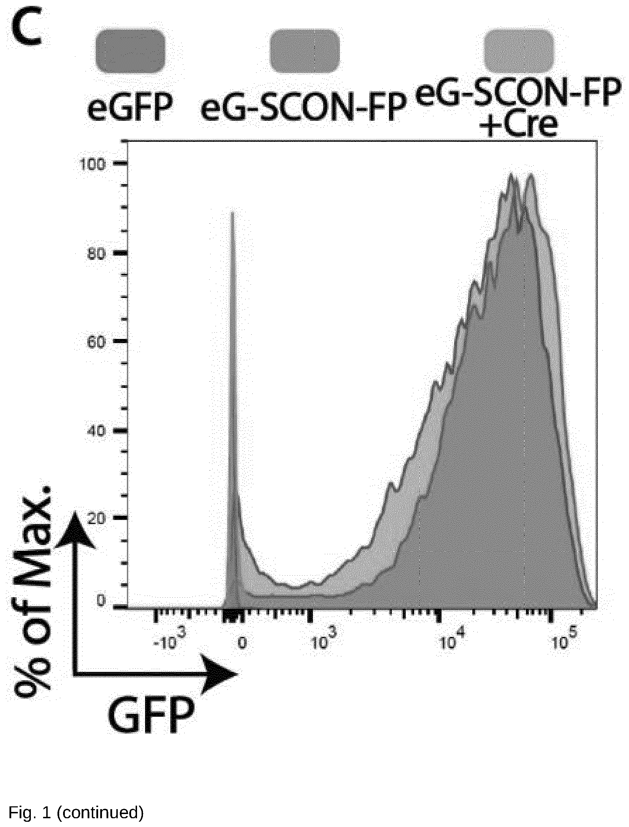

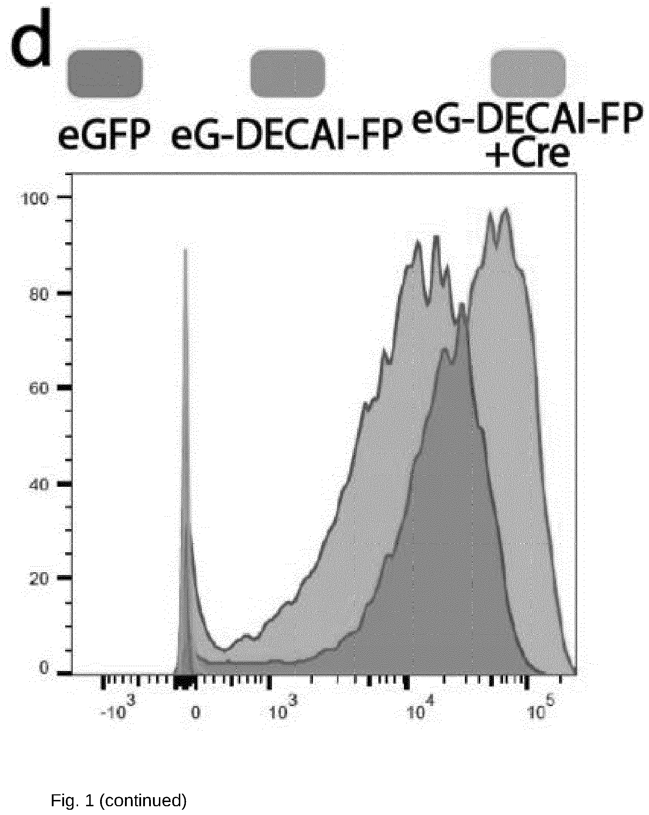

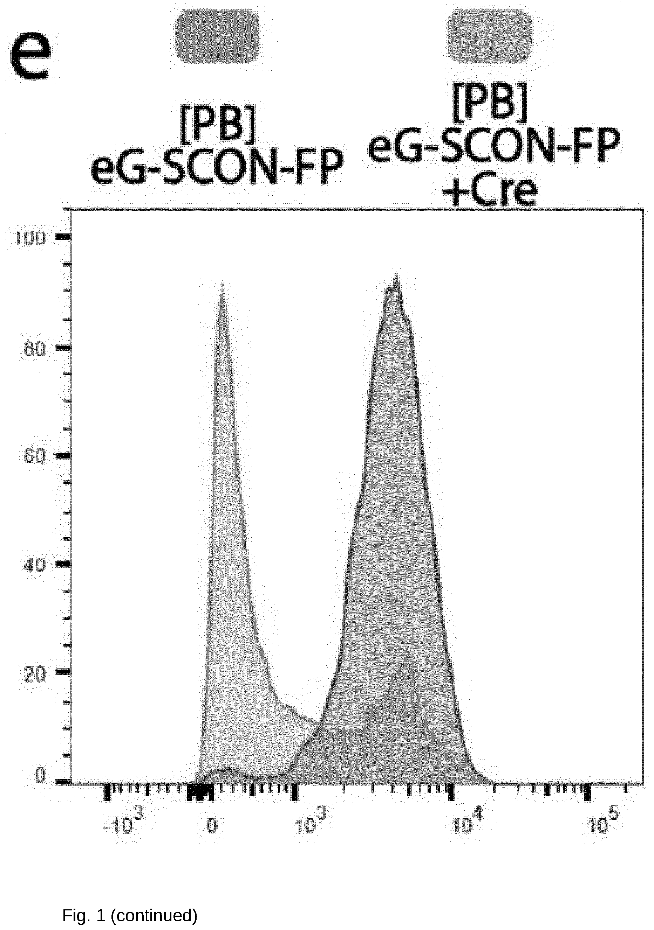

**Fig.1****. SCON functionality test with an eGFP overexpression construct. a,** Schematic drawing of SCON functionality test in an eGFP overexpression construct including intact eGFP, eG-SCON-FP and recombined eG-SCON-FP. SD, splice donor; BP, branch point; SA, splice acceptor. **b,** Images of transfected HEK293T cells on day 1, with intact eGFP, eG-SCON-FP and recombined eG-SCON-FP. Scale bar, 1mm. All constructures were co-transfected with a mCherry-overexpression plasmid. **c, d,** Histograms of the flow cytometry analysis of transfected HEK293T cells, comparisons between eGFP (red) and eG-SCON-FP (blue) **(c),** or between eGFP (red) and eG-DECAI-FP (blue) **(d),** and the respective recombined forms (yellow) **(c,d). e,** Flow cytometry analysis of mESCs with integrated piggybac-eG-SCON-FP transfected with Cre-expressing plasmid (yellow) or empty vector (blue).

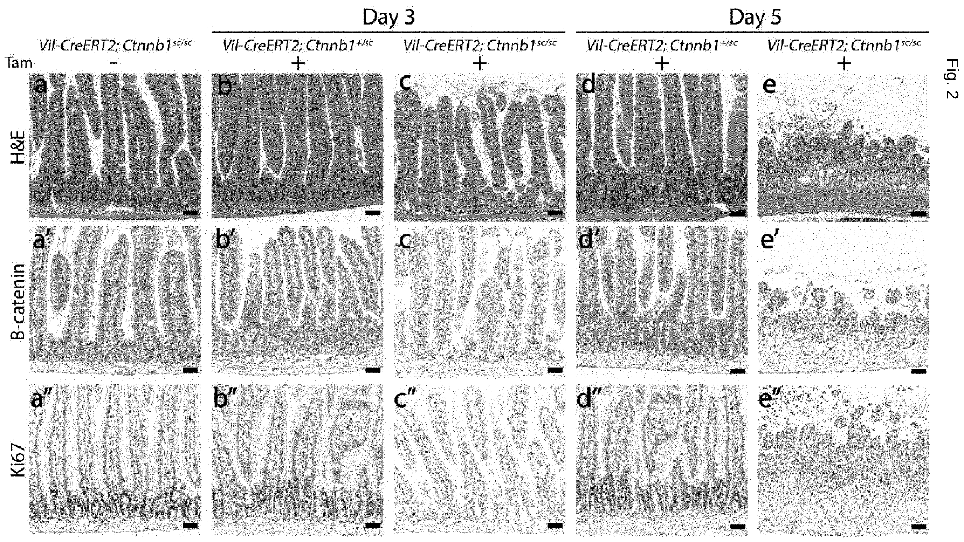

**Fig. 2****. Mouse *Ctnnb1^{sc}* is a functional conditional allele that works in *vivo.*** Homozygous (*Vil-CreER^{T2}; Ctnnb1*^{*sc*/*sc*}) intestines are healthy, with normal epithelial crypt-villus morphology **(a),** Beta-catenin on the cell membrane (**a'**) and Ki67 marking proliferating cells in the crypts (**a"**). Heterozygous (*Vil-CreER^{T2}; Ctnnb1*^{*+*/*sc*}) intestines are unaffected in morphology **(b, d),** Beta-catenin **(b', d')** and Ki67 (**b", d"**) after tamoxifen treatment. Homozygous mice treated with tamoxifen leads to loss of crypts on day 3 **(c),** Beta-catenin (**c'**) and Ki67 (**c"**) staining; and on day 5, the epithelium was completely lost (**e-e"**). H&E, hematoxylin and eosin. Scale bar, 50µm.

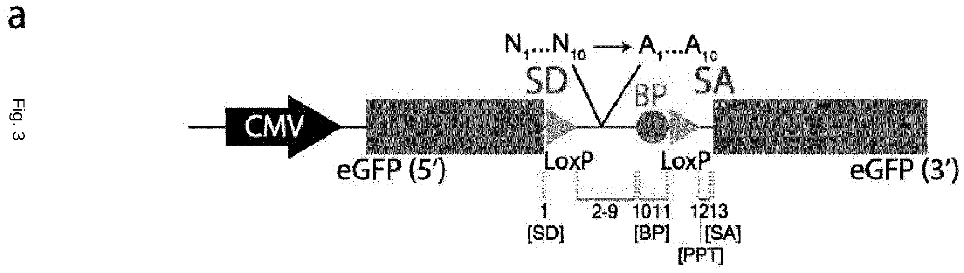

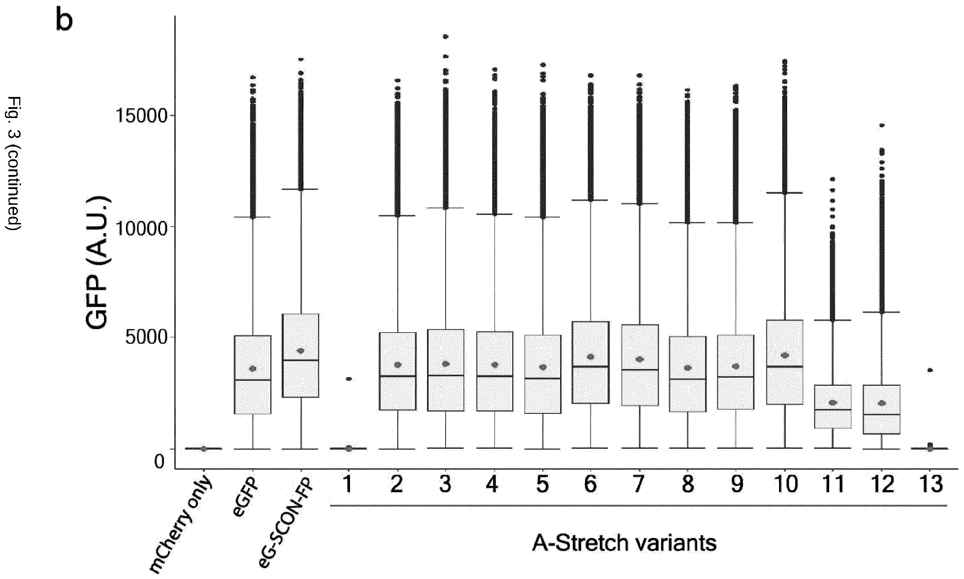

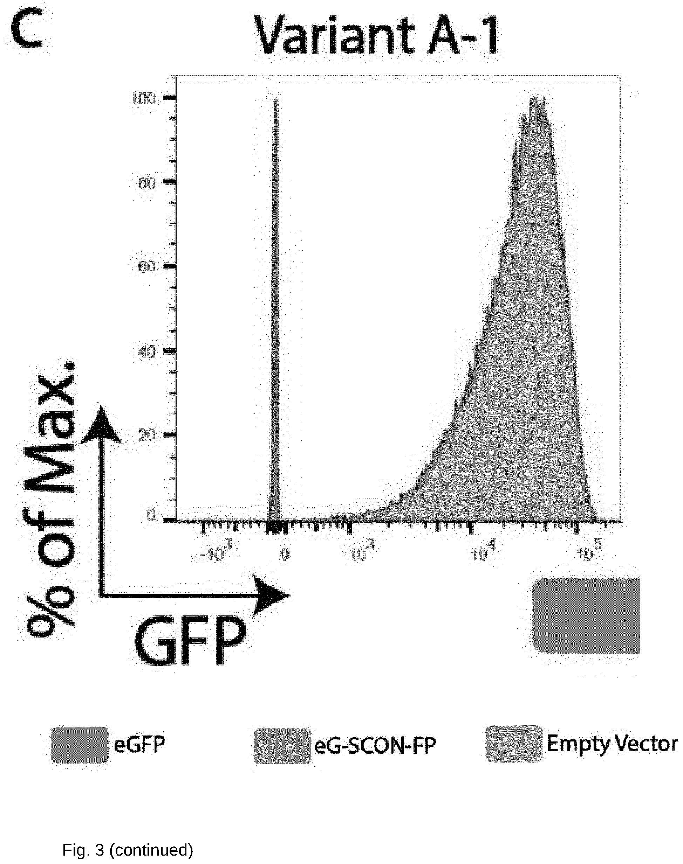

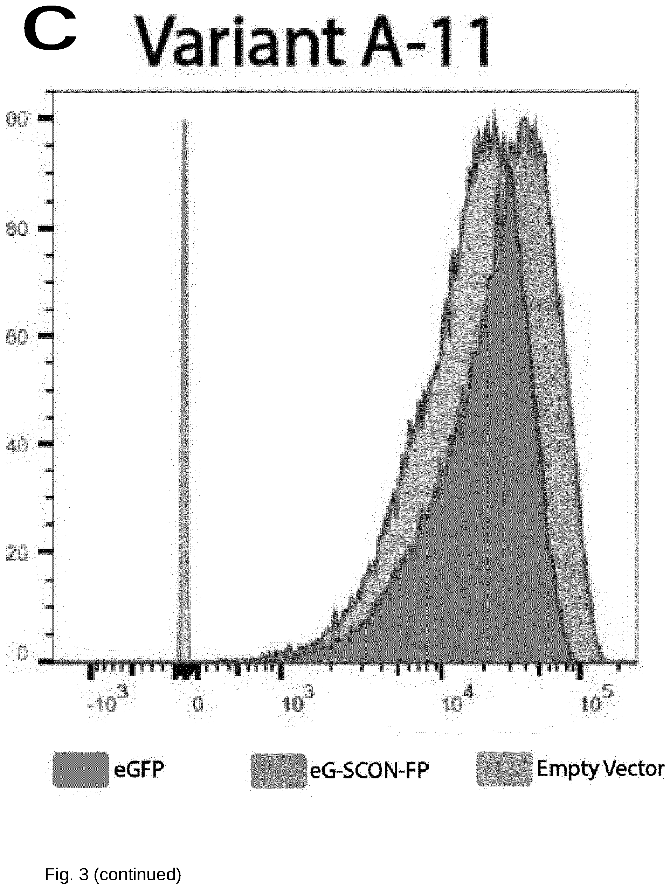

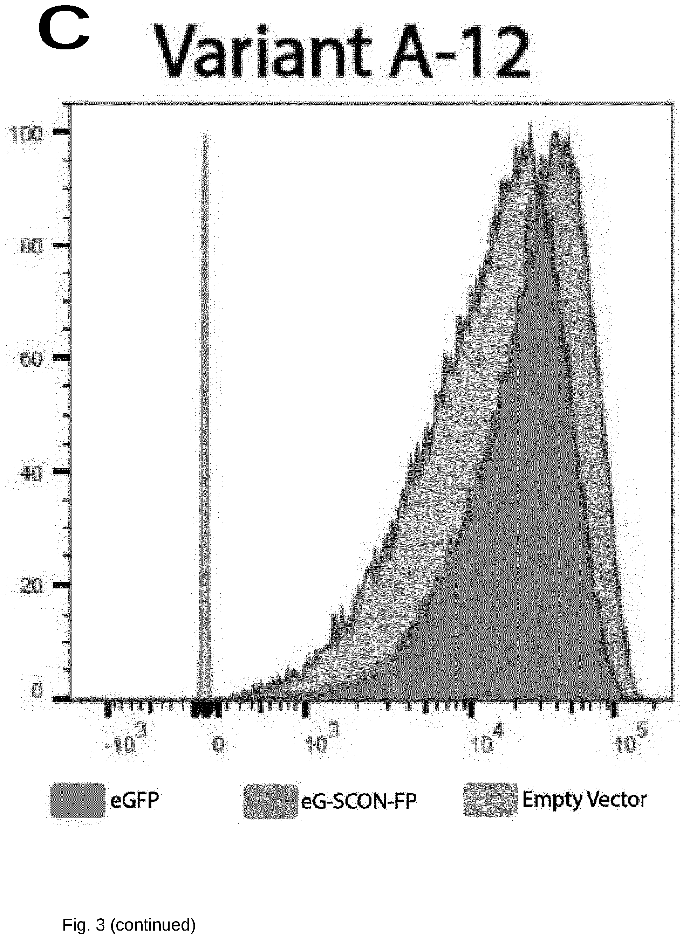

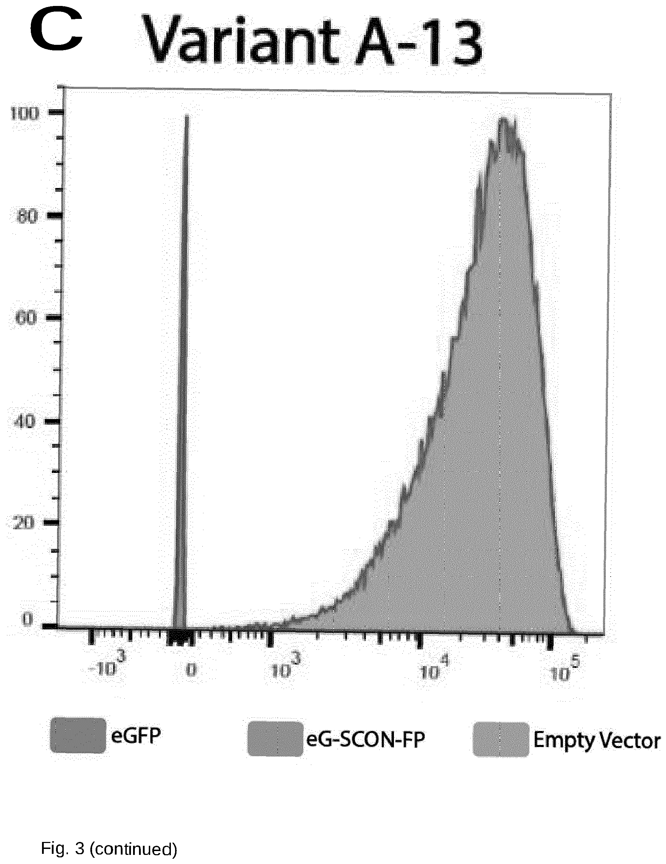

**Fig. 3****. Dissection of functional sequences within SCON cassette through A-stretch mutagenesis. a,** Schematic drawing of the 13 A-stretch variants, where 6-10 nucleotides are converted to adenine, which covers all sequences from SD to SA and excluding the LoxP sites. PPT, polypyrimidine tract. **b,** Boxplots of measured scaled values from flow cytometry analysis of HEK293T cells transfected with mCherry only, eGFP, eG-SCON-FP and the 13 A-stretch variants. The red dot indicates the median. **c,** Flow cytometry of HEK293T cells transfected with either variant A-1, A-11, A-12 and A-13 (blue) compared with intact eGFP (red) and empty vector or mCherry only (grey).

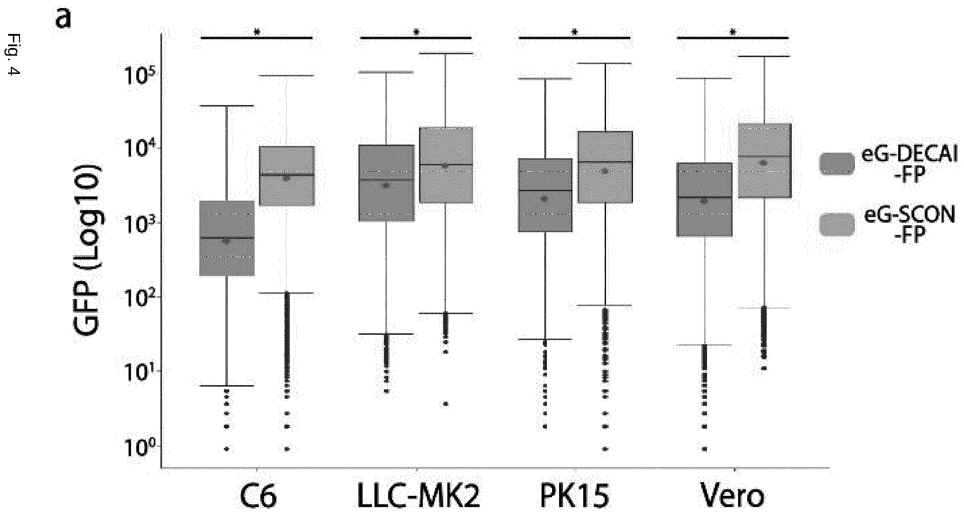

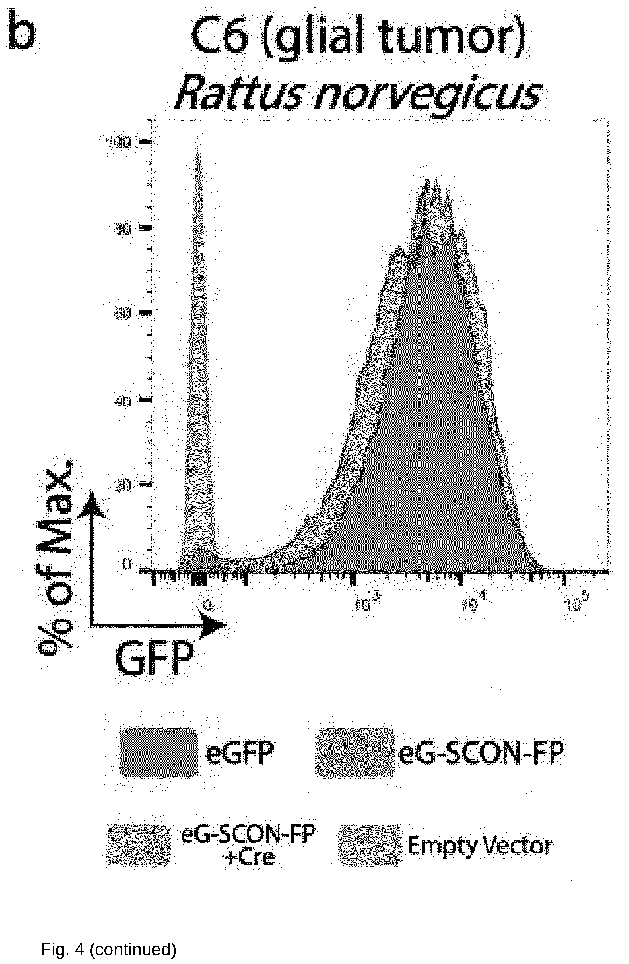

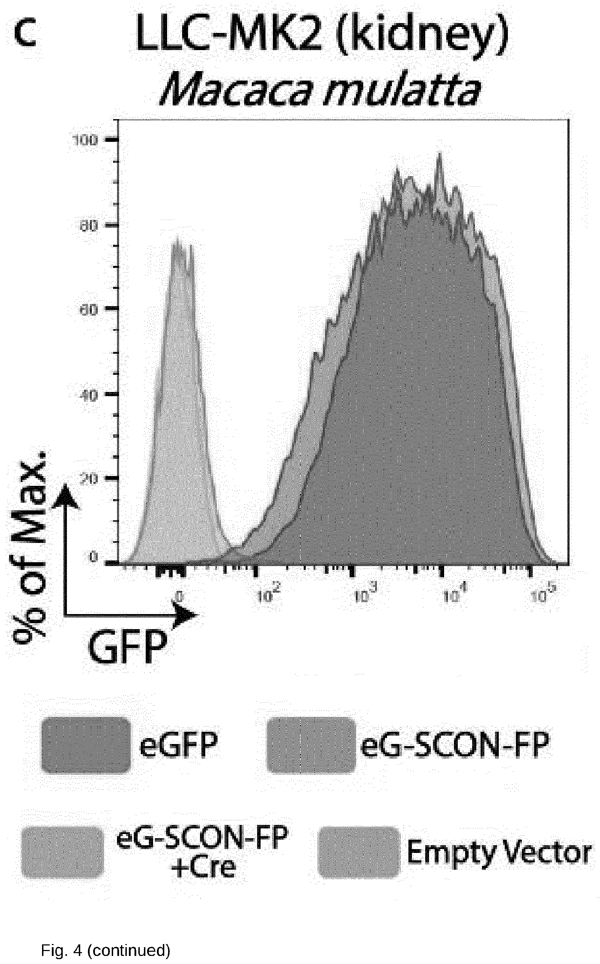

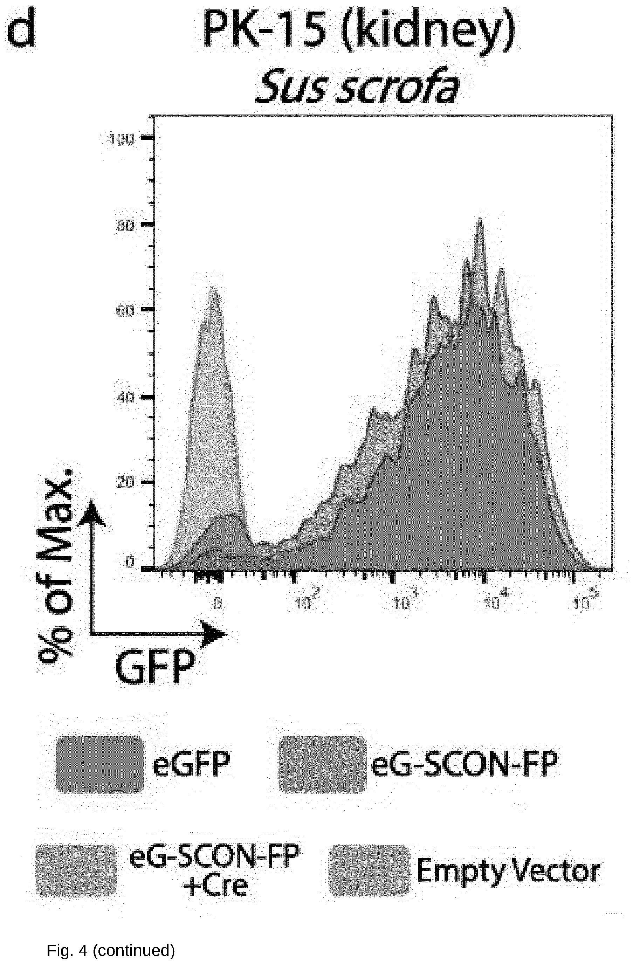

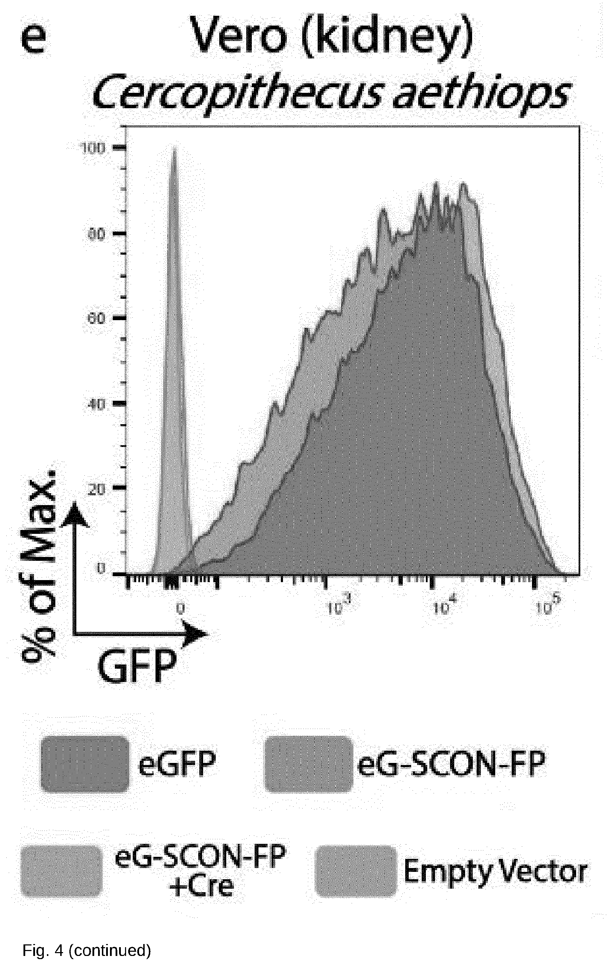

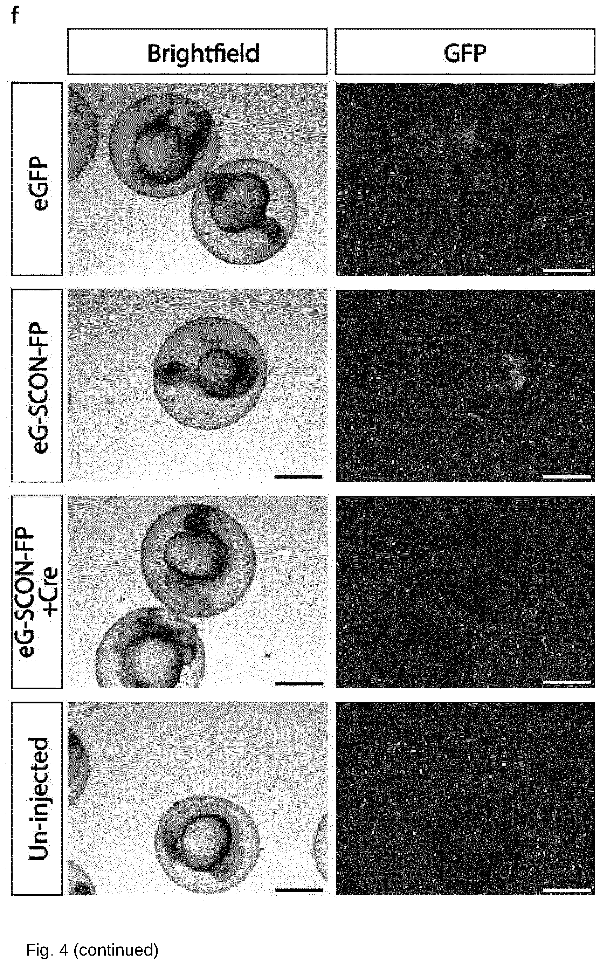

**Fig. 4****. SCON is applicable and neutral in various vertebrate species. a,** Flow cytometry analysis comparing the GFP levels in various cell lines transfected with eG-DECAI-FP (Grey) or eG-SCON-FP (Yellow). *p<0.001, from unpaired t-test. **b-e,** Flow cytometry analysis of C6 **(b),** LLC-MK2 **(c),** PK-15 **(d)** and Vero **(e)** cells transfected with either eGFP (red), eG-SCON-FP (blue), recombined eG-SCON-FP (yellow) or empty vector/ mCherry only (grey). **f,** Zebrafish embryos injected with eGFP, eG-SCON-FP or recombined eG-SCON-FP constructs, and un-injected controls analyzed 24 hours post fertilization. Scale bar, 500µm.

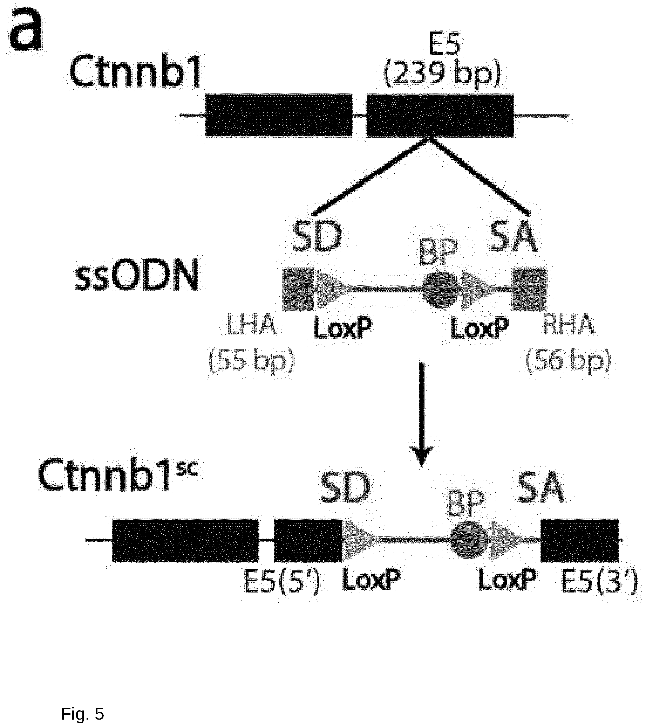

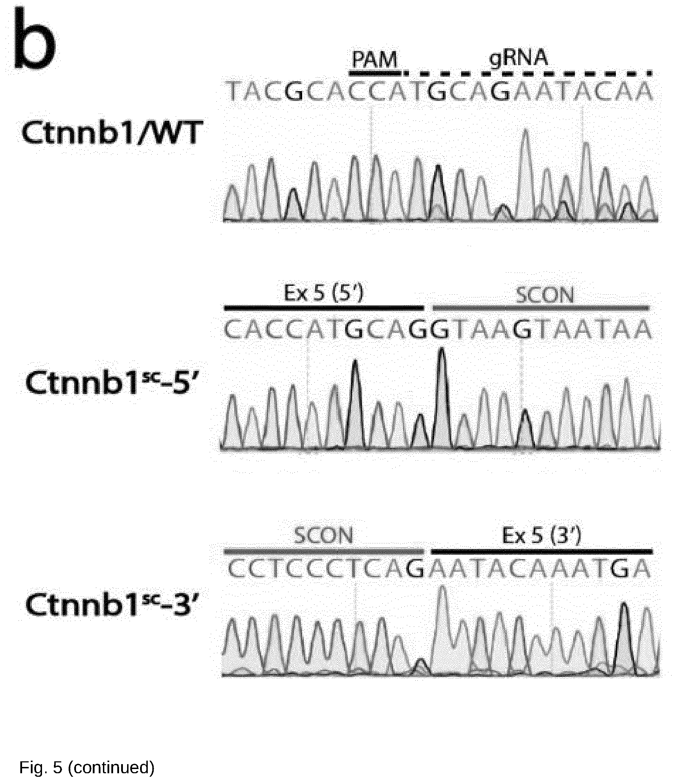

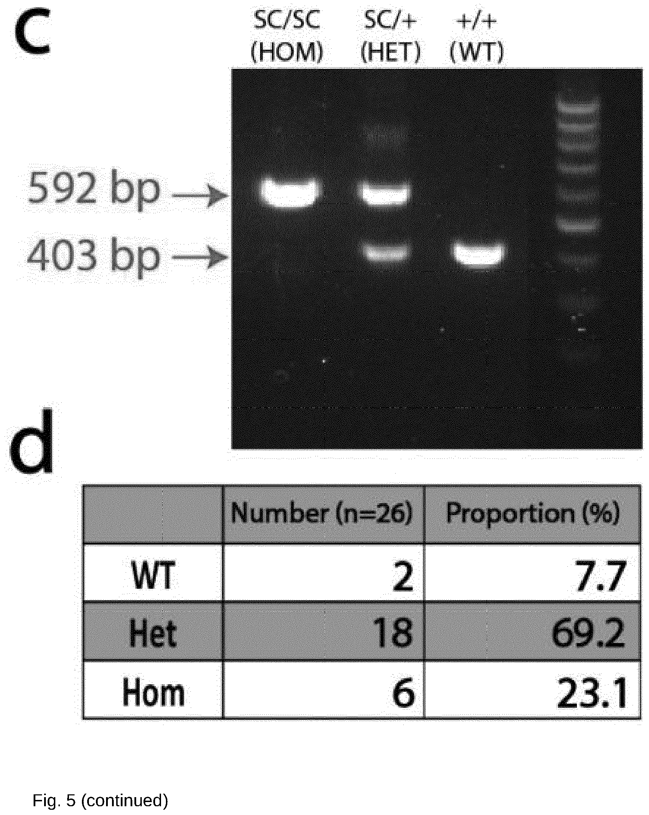

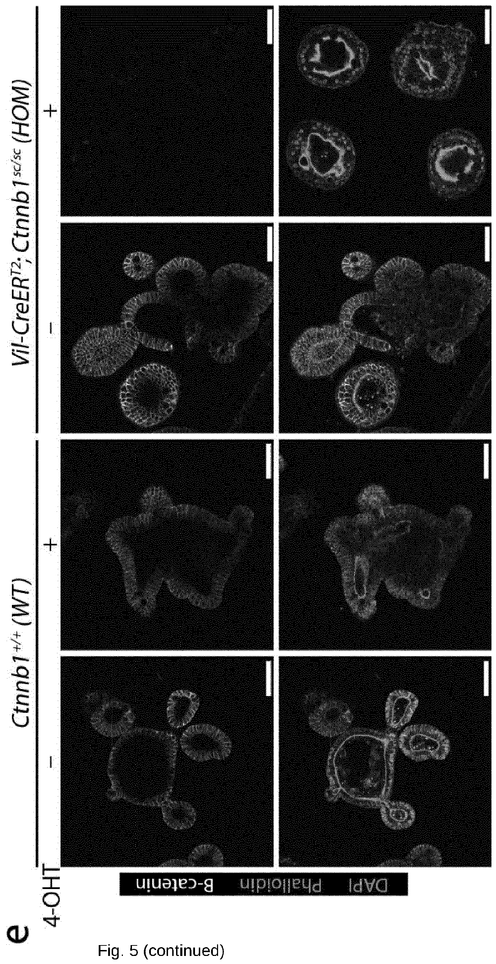

**Fig. 5****. Ctnnb1^{sc} mouse allele generated via one-step embryo injection and is tolerated at homozygosity.** a, Schematic drawing of SCON targeting ssODN, with 55 and 56 bp of left and right homology arms, respectively, into the exon 5 of Ctnnb1 gene. **b,** Sanger sequencing track of the WT, 5' and 3' alleles of Ctnnb1 and Ctnnb1^{sc}, respectively. **c,** Genotyping PCR of the *Ctnnb1*^{*sc*/*sc*} (HOM) , *Ctnnb1*^{*+*/*sc*} (HET) and *Ctnnb1*^{*+*/*+*} (WT), of which the lower (403 bp) and upper (592 bp) bands correspond to the WT and knock-in alleles, respectively. **d,** Genotype quantification from crossings of double heterozygotes (*Ctnnb1*^{*+*/*sc*})*.* Total number of offspring, n = 26. **e,** Small intestinal organoids from WT (*Ctnnb1*^{*+*/*+*}) and HOM (*Vil-CreER^{T2}; Ctnnb1*^{*sc*/*sc*}), treated with either 4-OH-tamoxifen (4-OHT) or vehicle for 8 hours. Organoids were fixed on day 4 and stained with Beta-catenin (grey), phalloidin (green) and Dapi (cyan). Scale bar, 100µm.

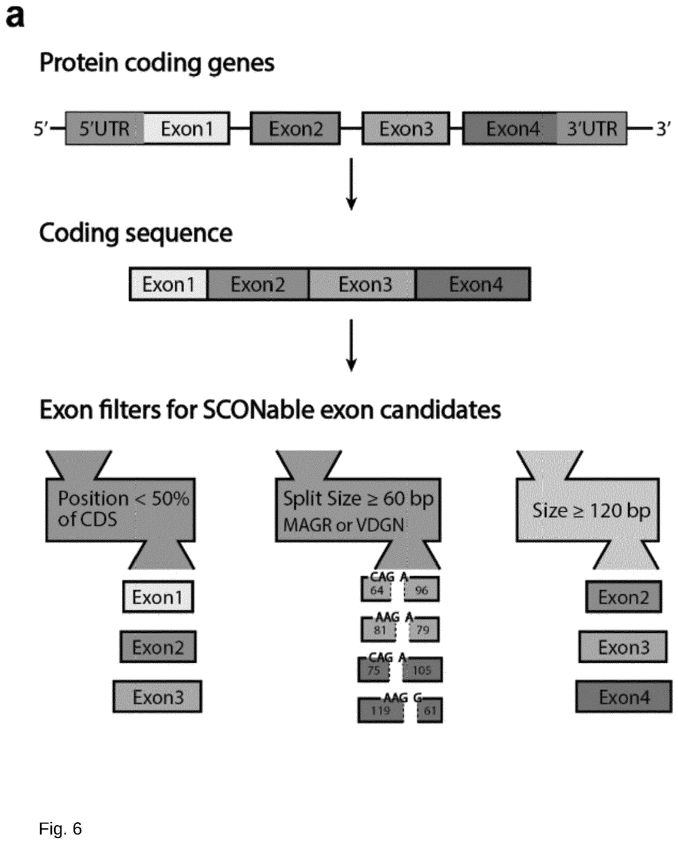

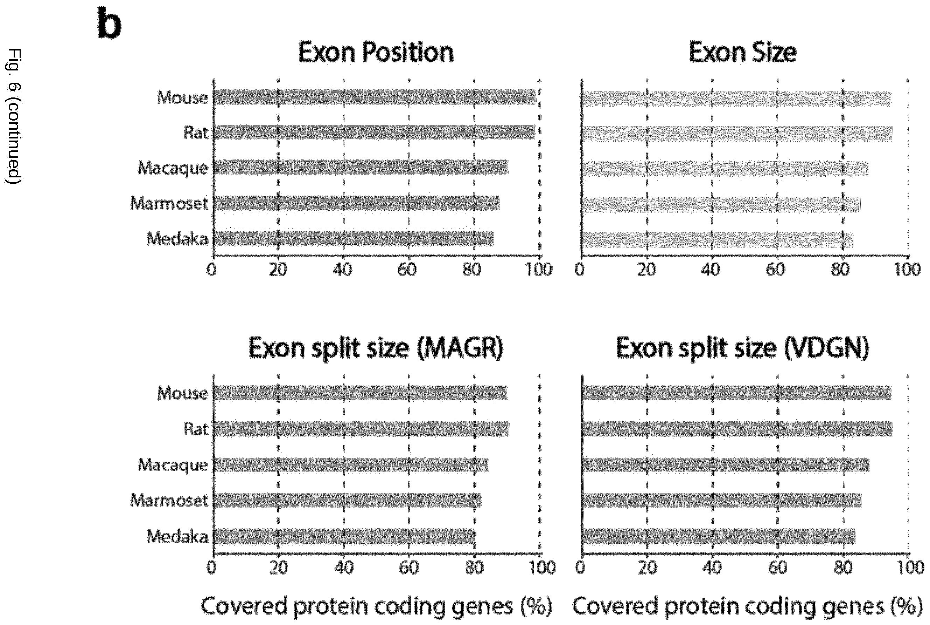

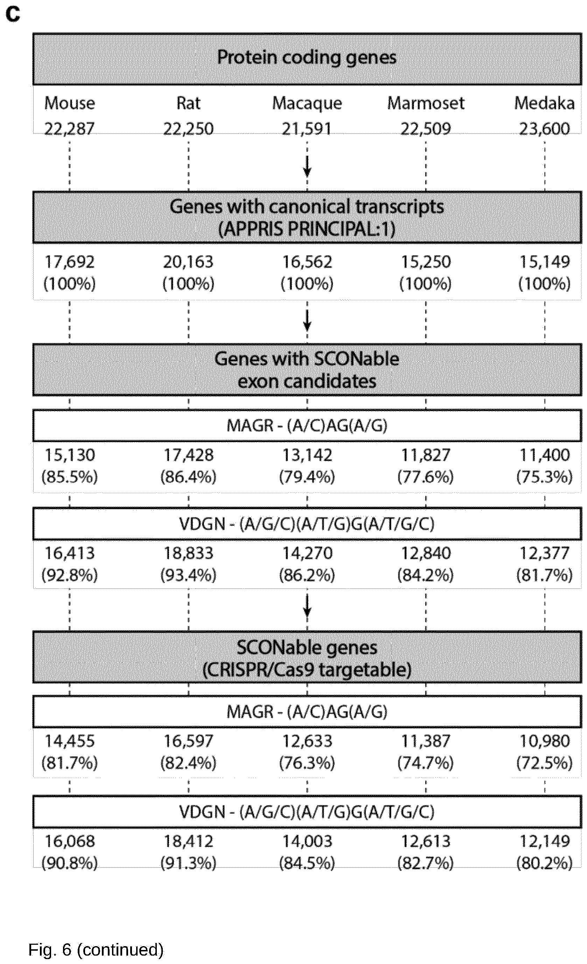

**Fig. 6****. Scheme for construction of database for SCON insertion sites. a,** Selection of the target exon candidates from protein coding transcripts. **b**, Gene coverages of individual exon filters such as position, size, and split exon size. **c,** Summary of databases for SCON insertion sites with CRISPR/Cas9 targeting sites.

### Examples

### Materials and methods

### Mice

All animal experiments were performed according to guidelines of the Austrian Animal Experiments Acts, and with valid project licenses which were approved by the Austrian Federal Ministry of Education, Science and Research and monitored by the institutional IMBA ethics and Biosafety department.

### Generation of Ctnnbl-SCON mouse

A *Ctnnb1-SCON* (Ctnnb1^{sc}) conditional KO mouse was generated via 2-cell stage embryo injection. To prepare the CRISPR injection mix, the following component was mixed together in 25µl with the final concentration in brackets: spCas9 mRNA (100ng/µl,), spCas9 protein (50 ng/µl), sgRNA (50 ng/µl) and ssODN (20ng /µl, Genscript). The mixture was spun down in a tabletop centrifuge at 4°C 13,000g for 15-20 minutes to prevent clogging of injection needles. Frozen 2-cell stage embryos of C57Bl/6JRj background (JANVIER LABS) were used for the cytoplasmic injection.

### Tamoxifen administration and organ harvest

Ctnnb1-SCON was crossed with Vil-CreER*^{T2}* (JAX, 020282, El Marjou et al. genesis 39, 186-193 (2004)) and bred to obtain either HET (Vil-CreER*^{T2}*; Ctnnb1^{+/sc}) or HOM (Vil-CreER*^{T2}*; Ctnnb1^{sc/sc}) mice. Tamoxifen (Sigma, T5648) dissolved in corn oil (Sigma, C8267) or corn oil only was injected intraperitoneally into 8-12 weeks old mice at final concentration of 3 mg Tamoxifen per 20 g body weight for CreER activation. Controlled mice received matched volume of corn oil. Uninduced, Day 3 or Day 5 mice were euthanized by cervical dislocation, and the intestines were harvested. The intestines were immediately cleaned with 1x PBS and flushed gently with 10% formalin solution (Sigma, HT501128), and fixed as 'swiss rolls' for 24 hours at room temperature. Fixed intestines were washed three times with 1x PBS, with 2-3 hours between each wash, before further processing.

### Genotyping

The toe clips or ear notches from the offspring were lysed in 30 µl of DirectPCR Lysis Reagent (Viagen) with 1µl of proteinase-K (20 mg/ml; Promega, MC5005) at 55°C overnight. The resulted mixture was diluted with 270 µl of nuclease-free water and spun down for at least 5 minutes in a tabletop centrifuge at 13,000 g. Then, 2-3 µl of the clear part of the solution was used for PCR reaction, with either Gotaq (Promega, M7808) or LongAmp 2X (NEB, M0287S). To check the sequences of genomic DNA, PCR bands at expected sizes were purified with a purification column.

### eGFP-SCON/ eGFP-DECAI constructs

The eGFP-SCON and eGFP-DECAI cassettes, where SCON or DECAI is inserted in the middle of eGFP, were synthesized and ordered from Genscript, and subsequently cloned into pcDNA4TO construct with BamHI (R0136S, NEB) and XhoI (R0146S, NEB) via ligation with T4 ligase (M0202S, NEB). The vectors were recombined with Cre-expressing bacteria (A111, Gene bridges) to obtain the recombined forms. The correct clones were confirmed with restriction digest SalI (R0138S, NEB) and Sanger sequencing.

### SCON A-stretch variants inserted in the eGFP cDNA

eGFP cDNA containing the SapI recognition sites at the selection intron insertion site, where the intron splice donor and acceptor was first cloned in the pcDNA4TO construct with BamHI and XhoI. Different SCON variant fragments containing the respective complementary ends were then inserted in the eGFP with SapI (R0569S, NEB) and T4 ligase for 20 cycles of 2 minutes at 37°C and 5 minutes at 16°C shuffling reaction. The mixture was transformed into *Escherichia coli* and DNA was extracted from individual colonies checked with restriction digests and Sanger sequencing.

### Cell culture and transfection

### HEK 293T cells

Human embryonic kidney (Hek) 293T cell culture was cultured in DMEM with high glucose with 10% fetal bovine serum (FBS, Sigma), 1% penicillin-streptomycin (P/S; Sigma, P0781) and 1% L-glutamine (L-glut; Gibco, 25030024).

### mES cells

Mouse ESCs (AN3-12) were cultured, as previously reported (Elling et al. Nature 550, 114-118 (2017)), in DMEM with high glucose (Sigma, D1152), 10% FBS (Sigma), 1% P/S, 1% L-glut, 1% NEAA (Sigma, M7145), 1% sodium pyruvate (Sigma, S8636), 0.1 mM 2-mercaptoethanol (Sigma, M7522), 7.5 µl of mouse LIF (stock concentration: 2 mg/ ml).

### Cell lines of other species

The following cell lines were cultured with medium supplemented with 10% FBS and 1% P/S, the corresponding basal medium are indicated in brackets: C6 (ATCC, CCL-107; DMEM-F12 (Gibco, 31330038)), PK15 (Elabscience Biotechnology, EP-CL-0187; Minimal essential medium (Gibco, 11095080), LLC-MK2 (Elabscience Biotechnology, ELSEP-CL-0141-1; RPMI-1640 (Sigma, R8758)), Vero (ATCC, CCL-81; DMEM-High glucose (Sigma, D1152)).

### Plasmid transfection

500,000-750,000 Cells were seeded in 6-well plates and left to attach and grow overnight. 2.5 µg of DNA (1ug of mCherry-expressing plasmid (Addgene, 72264), and 1.5 µg of pcDNA4TO-eGFP, -eG-SCON-FP, -eG-DECAI-FP or recombined forms of eG-SCON-FP or eG-DECAI-FP) was mixed with 8 µl of polyethyleneimine (1 mg/ ml, 23966) and incubated at room temperature for at least 15 minutes before being added dropwise to the cells. Culture media was exchanged 8-10 hours after transfection. 36 hours after transfection, cells were examined under the EVOS M7000 microscope (Thermo Scientific) with the brightfield, GFP and TexasRed filters. 36-48 hours after transfection, cells were dissociated into single cells for flow cytometry analysis, with a BD-LSRFor-tessa flow cytometer (BD). Data from the flow cytometry experiments were analyzed in FlowJo software (BD).

### Intestinal organoid culture

### Establishment and maintenance

Crypts were isolated from the proximal part of the small intestine as reported previously (Sato et al. Nature 459, 262-265 (2009)) and embedded in 15µl BME-R1 (R&D Systems, 3433010R1) droplets in a 48-well plate (Sigma, CLS3548-100EA). Organoids were established in WENR+Nic medium consisting of advanced Dul-becco's modified Eagle's medium/F12 (DMEM-F12; Gibco, 12634028) supplemented with pen/strep (100x; Sigma, P0781), 10 mM Hepes (Gibco, 15630056), Glutamax (100x; Gibco, 35050061), B27 (50x; Life Technologies, 17504044), Wnt3 conditioned medium (Wnt3a L-cells, 50% of final volume), 50 ng/ml recombinant mouse epidermal growth factor (EGF; Gibco, PMG8041), 100 ng/ml recombinant murine Noggin (PeproTech, 250-38), R-spondin-1 conditioned medium (HA-R-Spondin1-Fc 293T cells, 10% of final volume) and 10 mM nicotinamide (MilliporeSigma, N0636). For the first week of culture, primocin (InvivoGen, ant-pm-05) and ROCK-inhibitor/ Y-27632 (Sigma, Y0503) were supplemented to prevent microbial contamination and apoptosis, respectively. After the first passage, established organoids were converted to ENR budding organoid culture. Organoids were passaged with mechanical dissociation and diluted in 1:6 ratio.

### 4-Hydroxytamoxifen treatment

Budding organoids with passage 3 or higher were passaged with mechanical dissociation and seeded in BME droplets. Media containing vehicle (ethanol) or 500 nM 4-hydroxytamoxifen (Sigma, H7904) were added after BME polymerized. 8 hours after, media was exchanged back to ENR and replenished every two days.

### Histology and immunohistochemistry

Samples were processed using standard tissue protocol on Automatic Tissue Processor Donatello (Diapath). Samples were embedded in paraffin and were cut into 2µm sections onto glass slides. Hematoxylin and Eosin staining was done according to the standard protocol and using Gemini AS stainer (Thermo Scientific). For the immunohistochemistry following antibody staining, the following antibodies were used: Rabbit anti-Ki67 (1:200; 2 hours at room temperature; Abcam, Ab16667), Rabbit anti-β-catenin (1:300; 1 hour at room temperature; Abcam, ab32572). For signal detection, a two-step Rabbit polymer system HRP conjugated (DCS, PD000POL-K) was used. Stained slides were imaged with the 40x objective using the Pannoramic FLASH 250 III scanner (3DHISTECH) and images were cropped using the CaseViewer software.

### Immunofluorescence of intestinal organoids

BME droplets containing organoids were carefully collected into 1.5ml tubes and spun down in a tabletop centrifuge at 600g for 5 minutes. The supernatant and visible fraction of attached BME were removed. The pellet was resuspended in 4% paraformaldehyde and fixed at room temperature for 15-20 minutes. Fixed organoids were washed in 1xPBS for 3 times with 10-15-minute intervals. The organoids were blocked and permeabilized in a solution containing 5% DMSO, 0.5% Triton-X-100 (Sigma, T8787) and 2% normal donkey serum (Sigma, D9663) for one hour at 4°C. Then, the samples were stained overnight with Alexa 647-conjugated mouse anti-β-catenin (1:200; Cell Signaling Technology, 4627S), ATTO 488-conjugated phalloidin (1:300; Sigma, 49409-10NMOL). The samples were washed three times with 1xPBS and during the last wash, the samples were incubated with 2µg/ ml DAPI and mounted onto coverslips in a solution containing 60% glycerol and 2.5M fructose (Dekkers et al. Nature Protocols 14, 1756-1771 (2019)). The imaging was done with a multiphoton SP8 confocal microscope (Leica).

### Computing SCON targetable sites

In order to construct databases for SCON insert sites, we used genomic information, about sequence, exon, coding region, and gene type, derived from Ensembl Biomart, build 102 (www.ensembl.org/, Yates et al., Nucleic Acids Research, 2019, Ensembl 2020) for mouse (*M. musculus*)*,* rat (*R. norvegicus*)*,* macaque (M. *mulatta*)*,* marmoset (C. *jacchus*)*,* and medaka (*O*. *latipes*)*.* In order to map canonical transcripts to genes, we derived 'PRINCIPAL:1' from APPRIS database for mouse and rat with Ensembl, build 102 (appris-tools.org, Rodriguez, J. M. et al. Nucleic Acids Res. 46, D213-D217 (2018)) and we regard the longest transcript as canonical transcript for other species. The quality features including GC-content, self-complementarity, and mismatch scores for each the candidate sites are mapped by same approach as CHOPCHOP (chopchop.cbu.uib.no, Labun, K. et al. Nucleic Acids Res. 47, W171-W174 (2019).

### Example 1: SCON is a versatile conditional intron without discernable hypomorphic effects

This example shows the generation and use of a Short Conditional intrON (SCON) cassette that shows no hypomorphic effect in various vertebrate species and is compatible for targeting via one-step zygote microinjections.

The generation of conditional allele using CRISPR technology is still challenging. As best mode a SCON of 189 bp in size is used to enable a rapid generation of conditional allele with one-step zygote injection. SCON has conditional intronic function in various vertebrate species and its target insertion is as simple as CRISPR/Cas9-mediated gene tagging.

The SCON shown here for illustration purposes is a modified intron derived from the first intron (130 bp) of human *HBB* (Hemoglobin subunit beta) gene. In complete form, this SCON is 189 bp long, consisting of, from the 5' to 3' end, a splice donor, LoxP recombination site 1, a branch point, LoxP recombination site 2, polypyrimidine tract, and a splice acceptor. By design, it has a similar sequence architecture to the previously introduced conditional intronic system - DECAI (201 bp, Guzzardo et al. Scientific Reports 7, (2017)), which however showed a hypomorphic effect at the level of protein expression.

To optimize conditional intron function, several features were implemented into SCON, including: 1) the optimized length between the putative branch point and the splice acceptor is kept at 45 bp to allow efficient splicing to take place, 2) 100 bp distance was used between the two LoxP sites for an efficient Cre-LoxP recombination, and 3) other miscellaneous changes were incorporated for optimal splice donor, acceptor and pyrimidine tract sequences. Upon recombination, SCON is reduced to 55 bp, of which all three reading frames contain translational stop codons within the remaining LoxP sequence such that gene loss of function occurs via premature translational termination.

### DECAI: comparative insert (Guzzardo et al.):

**GTAAG**TAATAACTTCGTATAGCATACATTATACGAAGTTATTCAAGGTTAGAAGACAGGTTTAA GGAGACCAATAGAAACTGGGCTTGTCGAGACAGAGAAGACTCTTGCGTTT**CTGAT**AGGCACCTA TTGGTCTTA**CTGAC**ATCCACTTTGCCATAACTTCGTATAGCATACATTATACGAAGTTATTTTC TCTCCAC**AG** (SEQ ID NO: 1) highlighted elements from 5' to 3': bold: splice donor; underlined: loxP site; 2x bold: 2x branch point; underlined: loxP site; bold: splice acceptor

### Optimized SCON (189 bp):

**GTAAG**TAATAACT TCGTATAAGGTATCCTATACGAAGTTATTCTCTCTGCCTATTGG GGTTACA AGACAGGTTTAAGGAGACCAATAGAAACTGGGCATGTGGAGACAGAGAAGACTCTTGGGTTT**CT GAT**AGGCA**CTGAC**ATAACTTCGTATAAGGTATCCTATACGAAGTTATTTTCCCTCCCTC**AG** (SEQ ID NO: 2) highlighted elements from 5' to 3': bold: splice donor; underlined: loxP site; 2x bold: 2x branch point; underlined: loxP site; bold: splice acceptor

The optimized SCON is cloned from a nucleic acid with the sequence:

TAGGCTTGTCCCGTTTCCACAGGGCTCTTCTAAGGTAAGTAATAACTTCGTATAAGGTATCCTA
TACGAAGTTATTCTCTCTGCCTATTGGGGTTACAAGACAGGTTTAAGGAGACCAATAGAAACTG
GGCATGTGGAGACAGAGAAGACTCTTGGGTTTCTGATAGGCACTGACATAACTTCGTATAAGGT
ATCCTATACGAAGTTATTTTCCCTCCCTCAGGacAGAAGAGCGGGCAACTTGCCCCATCCAGTG
G (SEQ ID NO: 3)

Firstly, we validated the functionality of SCON in an eGFP overexpression construct. We co-transfected mCherry cassette (serving as transfection control) with a cassette containing either the intact eGFP, eG-SCON-FP or already Cre-recombined eG-SCON-FP (Fig. 1a) into HEK293T cells, and assessed the fluorescent level by fluorescent microscope and flow cytometry (Fig. 1b, c). We also carried out the same test in parallel with DECAI for comparisons. Both eG-SCON-FP and eG-DECAI-FP showed GFP expression, whereas the recombined forms had no detectable fluorescence (Fig. 1b-d). Interestingly, eG-SCON-FP exhibited similar level of GFP signals compared to the intact eGFP construct (Fig. 1c). However, eG-DECAI-FP showed reduced levels (Fig. 1d), indicating an adverse hypomorphic effect as an intron, which is in-line with previous observation (Guzzardo et al.). Thus, SCON showed a reliable conditional intronic function with no discernable hypomorphic effect.

To understand better the functionality of different parts within the SCON cassette, we designed a series of "A-stretch variants" where 6-10 nt within SCON is converted into adenine (Fig. 3a).

A-stretch variants (A-stretch in comparison to SEQ ID NO: 2 highlighted in bold):
(1) SCON100-LoxP-A1-6 
AAAAAAAATAACTTCGTATAAGGTATCCTATACGAAGTTATTCTCTCTGCCTATTGGGGTTACA
AGACAGGTTTAAGGAGACCAATAGAAACTGGGCATGTGGAGACAGAGAAGACTCTTGGGTTTCT
GATAGGCACTGACATAACTTCGTATAAGGTATCCTATACGAAGTTATTTTCCCTCCCTCAG
(SEQ ID NO: 4)
(2) SCON100-LoxP-A42-51 
GTAAGTAATAACTTCGTATAAGGTATCCTATACGAAGTTATAAAAAAAAAATATTGGGTTACA
AGACAGGTTTAAGGAGACCAATAGAAACTGGGCATGTGGAGACAGAGAAGACTCTTGGGTTTCT
GATAGGCACTGACATAACTTCGTATAAGGTATCCTATACGAAGTTATTTCCCTCCCTCAG
(SEQ ID NO: 5)
(3) SCON100-LoxP-A52-61 
GTAAGTAATAACTTCGTATAAGGTATCCTATACGAAGTTATTCTCTCTGCCAAAAAAAAAAACA
AGACAGGTTTAAGGAGACCAATAGAAACTGGGCATGTGGAGACAGAGAAGACTCTTGGGTTTCT
GATAGGCACTGACATAACTTCGTATAAGGTATCCTATACGAAGTTATTTTCCCTCCCTCAG
(SEQ ID NO: 6)
(4) SCON100-LoxP-A62-71 
GTAAGTAATAACTTCGTATAAGGTATCCTATACGAAGTTATTCTCTCTGCCTATTGGGGTTAAA
AAAAAAATTTAAGGAGACCAATAGAAACTGGGCATGTGGAGACAGAGAAGACTCTTGGGTTTCT
GATAGGCACTGACATAACTTCGTATAAGGTATCCTATACGAAGTTATTTTCCCTCCCTCAG
(SEQ ID NO: 7)
(5) SCON100-LoxP-A72-81 
GTAAGTAATAACTTCGTATAAGGTATCCTATACGAAGTTATTCTCTCTGCCTATTGGGGTTACA
AGACAGGAAAAAAAAAACCAATAGAAACTGGGCATGTGGAGACAGAGAAGACTCTTGGGTTTCT
GATAGGCACTGACATAACTTCGTATAAGGTATCCTATACGAAGTTATTTCCCTCCCTCAG
(SEQ ID NO: 8)
(6) SCON100-LoxP-A82-91 
GTAAGTAATAACTTCGTATAAGGTATCCTATACGAAGTTATTCTCTCTGCCTATTGGGGTTACA
AGACAGGTTTAAGGAGAAAAAAAAAAACTGGGCATGTGGAGACAGAGAAGACTCTTGGGTTTCT
GATAGGCACTGACATAACTTCGTATAAGGTATCCTATACGAAGTTATTTTCCCTCCCTCAG
(SEQ ID NO: 9)
(7) SCON100-LoxP-A92-101 
GTAAGTAATAACTTCGTATAAGGTATCCTATACGAAGTTATTCTCTCTGCCTATTGGGGTTACA
AGACAGGTTTAAGGAGACCAATAGAAAAAAAAAAAAAGGAGACAGAGAAGACTCTTGGGTTTCT
GATAGGCACTGACATAACTTCGTATAAGGTATCCTATACGAAGTTATTTTCCCTCCCTCAG
(SEQ ID NO: 10)
(8) SCON100-LoxP-A102-111 
GTAAGTAATAACTTCGTATAAGGTATCCTATACGAAGTTATTCTCTCTGCCTATTGGGGTTACA
AGACAGGTTTAAGGAGACCAATAGAAACTGGGCATGTAAAAAAAAAAAGACTCTTGGGTTTCT
 
GATAGGCACTGACATAACTTCGTATAAGGTATCCTATACGAAGTTATTTTCCCTCCCTCAG
(SEQ ID NO: 11)
(9) SCON100-LoxP-A112-121 
GTAAGTAATAACTTCGTATAAGGTATCCTATACGAAGTTATTCTCTCTGCCTATTGGGGTTACA
AGACAGGTTTAAGGAGACCAATAGAAACTGGGCATGTGGAGACAGAGAAAAAAAAAAGGTTTCT
GATAGGCACTGACATAACTTCGTATAAGGTATCCTATACGAAGTTATTTCCCTCCCTCAG
(SEQ ID NO: 12)
(10) SCON100-LoxP-A122-131 
GTAAGTAATAACTTCGTATAAGGTATCCTATACGAAGTTATTCTCTCTGCCTATTGGGGTTACA
AGACAGGTTTAAGGAGACCAATAGAAACTGGGCATGTGGAGACAGAGAAGACTCTTGAAAAAAA
AAAAGGCACTGACATAACTTCGTATAAGGTATCCTATACGAAGTTATTTCCCTCCCTCAG
(SEQ ID NO: 13)
(11) SCON100-LoxP-A132-141 
GTAAGTAATAACTTCGTATAAGGTATCCTATACGAAGTTATTCTCTCTGCCTATTGGGGTTACA
AGACAGGTTTAAGGAGACCAATAGAAACTGGGCATGTGGAGACAGAGAAGACTCTTGGGTTTCT
GATAAAAAAAAAAAATAACTTCGTATAAGGTATCCTATACGAAGTTATTTCCCTCCCTCAG
(SEQ ID NO: 14)
(12) SCON100-LoxP-A176-185 
GTAAGTAATAACTTCGTATAAGGTATCCTATACGAAGTTATTCTCTCTGCCTATTGGGGTTACA
AGACAGGTTTAAGGAGACCAATAGAAACTGGGCATGTGGAGACAGAGAAGACTCTTGGGTTTCT
GATAGGCACTGACATAACTTCGTATAAGGTATCCTATACGAAGTTATAAAAAAAAAATCAG
(SEQ ID NO: 15)
(13) SCON100-LoxP-A185-189

GTAAGTAATAACTTCGTATAAGGTATCCTATACGAAGTTATTCTCTCTGCCTATTGGGGTTACA
AGACAGGTTTAAGGAGACCAATAGAAACTGGGCATGTGGAGACAGAGAAGACTCTTGGGTTTCT
GATAGGCACTGACATAACTTCGTATAAGGTATCCTATACGAAGTTATTTTCCCTCCCAAAA
(SEQ ID NO: 16)

Out of the 13 different variants, we found that four variants had either hypomorphic or complete reduction of eGFP fluorescence when inserted as an intron (Fig. 3b, c). These included a branch point (variant 11) and polypyrimidine tract (variant 12) that resulted in hypomorphic expression of the inserted eGFP cassettes; whereas mutating the splice donor (variant 1) and splice acceptor (variant 13) resulted in complete loss of eGFP level (Fig. 3b, c). These data suggest that the elements splice donor, spice acceptor and the branch point closer to the splice acceptor needed for optimal intronic function of SCON and most parts between the two LoxP sites are not as essential. Removal of the branch point farther away from the splice acceptor (variant 10) had no negative effect, meaning that the hypomorphic effect can be avoided by maintaining the branch point closer to the splice acceptor. Only one branch point is needed.

Next, we tested whether SCON can be efficiently recombined in mammalian cells. We cloned the eG-SCON-FP construct into a PiggyBac transposon backbone, and generated a clonal mouse embryonic stem (ES) cell line with constitutive eGFP expression containing SCON. By co-transfecting Cre-expressing plasmid with mCherry cassette into eG-SCON-FP-expressing ES cells, we observed an efficient reduction of eGFP level within the mCherry+ population whereas mock control (transfected only with mCherry cassette) maintained high levels of eGFP (Fig. 1e). Taken together, our data indicate the suitability of SCON to be utilized in mammalian system as conditional intron, where SCON is neutral upon insertion and can be efficiently recombined by Cre recombinase to abolish its intronic function and cause knockout of the inserted gene.

### Example 2: Neutrality of SCON is conserved in various species

cKO alleles have been widely used in mice for decades thanks to the robust set up with germline competent ES cells (Mulas et al. Development 146, dev173146 (2019)), the endeavor of International Knockout Mouse Consortium, and also recently via the advent of CRISPR technology (Quadros et al. Genome Biology 18, (2017)). However, for many other vertebrate species such as zebrafish, rat, porcine, bovine and non-human primate models, the use of cKO approach has been limited largely due to the lack of reliable germline competent ES cells. We sought to test whether SCON would be a suitable cKO strategy for other species. Therefore, we made use of cell lines of different species, including C6 (*Rattus norvegicus*)*,* PK15 (*Sus scrofa*)*,* LLC-MK2 (*Macca mulatta*) and Vero (*Cercopithecus aethiops*) and transfected them with overexpression constructs of eGFP, eG-SCON-FP or eG-DECAI-FP and the corresponding Cre-recombined forms. In line with the results in HEK293T and mES cells, SCON intron did not show any discernable hypomorphic effect in all tested cell lines (Fig. 4b-e, in blue), while the recombined forms of SCON abrogated the eGFP expression completely (Fig.4 b-e, in orange). Intriguingly, in three of the cell lines (C6, PK15 and Vero), eG-DECAI-FP showed reduced levels of eGFP compared to eG-SCON-FP (Fig. 4a), again showing that SCON is devoid of the hypomorphic effects. In addition, we also explored the possibility of utilizing SCON in zebrafish by injecting the eGFP, eG-SCON-FP and the Cre-recombined form into the fertilized egg. We examined the embryos 24 hours post fertilization and observed that eGFP and eG-SCON-FP expressed well detectable eGFP fluorescence whereas the embryos having the recombined form did not (Fig. 4f). This indicates that SCON has the potential to be utilized as cKO systems in various vertebrate species.

### Example 3: Targeted insertion of SCON via one-step zygote injection

As previous in vitro overexpression-based system highlighted the desired features of SCON for cKO approaches, we sought to test whether SCON would also work well in targeting endogenous genes. Therefore, we chose to generate SCON cKO *Ctnnb1* allele (Fig. 5a), which encodes for beta-catenin and is a developmentally required gene with early lethality upon knockout in mice. We injected CRISPR ribonucleoprotein (RNP) with 300 bp-long, company-synthesized, single-stranded deoxynucleotides (ssODNs) consisting of SCON with short homology arms (55 and 56 bp for the 5' and 3' homology arms, respectively) into the cytoplasm of the developing 2-cell stage mouse embryos (Gu et al. Nature Biotechnology 36, 632-637 (2018)). We detected one out of thirteen offspring (7.7%), having a precise heterozygous integration with the other allele remaining intact (Fig. 5b). From this offspring we could backcross to confirm germline transmission and bred to homozygosity (*Ctnnb1*^{*sc*/*sc*}) (Fig. 5c). From the heterozygote to heterozygote (HET) crosses, we have not observed under-represented ratios of the homozygote (HOM) mice (Fig. 5d) and those HOM mice showed no discernible phenotype, confirming the intact gene function with SCON insertion.

To verify the conditional functionality of *Ctnnb1^{sc} allele,* we utilized the *Villin-CreER ^{T2}* (*Vil-CreER^{T2}*) for intestinal epithelium-specific Cre recombination. We first isolated crypts from the duodenum of HOM (*Vil-CreER^{T2}; Ctnnb1*^{*sc*/*sc*}) and wildtype (*Ctnnb1*^{+/+}) mice to establish adult stem cell (AdSC)-based intestinal organoids. Then, transient 4-Hydroxytamoxifen (4-OHT) treatment for 8 hours was carried out in budding organoid culture (with Egf, Noggin and R-spondin). Both 4-OHT treated and untreated wildtype organoids and untreated HOM organoids grew normally (Fig. 5e). However, 4-OHT treated HOM organoids halted in growth or collapsed on day 2 onward (Fig. 5e). We confirmed the loss of beta-catenin by immunostaining, while DAPI and phalloidin staining shows live cells in those small cystic mutant organoids (Fig. 5e).

To directly verify the functionality *in vivo,* we injected 3 mg Tamoxifen (TAM) per 20 g body weight into both HOM *(Vil-CreER ^{T2}*; *Ctnnb1*^{sc/sc}) and HET *(Vil-CreER ^{T2}; Ctnnb1*^{+/sc}) mice, and harvested the intestines on day 3 and 5. Control samples showed normal crypt-villus axis, detectable membrane-bound beta-catenin, and Ki67+ proliferating zone in the bottom of the crypts where stem and progenitor cells are located (Fig. 2a-a", b-b", d-d"). On day 3, TAM-treated HOM samples showed clear loss of proliferative crypts with the loss of beta-catenin staining (Fig. 2c-c"). On day 5, TAM-treated HOM mice showed shortened and inflamed intestines, sections of which showed nearly entire loss of intestinal epithelium (Fig. 2e-e"), thus indicating efficient recombination of *Ctnnb1*^{sc} allele *in vivo* and the loss of beta-catenin function upon recombination.

### Example 4: SCON is applicable in large fraction of protein-coding genes

To systematically estimate optimal sites for easy access for SCON integration, we carried out bioinformatic analysis to screen for insertion sites in mouse, rat, macaque, marmoset and medaka genomes (Fig. 6). The selection criteria include: 1) target exons are positioned within the first 50% of the protein coding-sequence from canonical transcripts of protein-coding genes; 2) intron insertion sites contain either stringent (MAGR, (A/C)-A-G-(A/G), Burset et al. Nucleic Acids Res. 28, 4364-4375 (2000)) or flexible (VDGN, (A/G/C)-(A/T/G)-G-(A/T/G/C), Ma et al. PLoS One 10, 1-12 (2015)) splice junction consensus sequences; 3) exon should be larger than 120bp in length and that both 5' and 3' split exons to be at least 60bp (Fig. 6a; Andersson-Rolf et al. Nat. Methods 14, 287-289 (2017)). After combining all selection criteria, we identified that majority of coding genes are optimal targets in all five species, on average 80.8% for MAGR and 87.7% for VDGN intron insertion sites (Fig. 6b, c). We also found CRISPR/Cas9 targeting stie(s) around intron insertion sites in most cases (Fig. 6c). This is a conservative estimate as some genes with an important domain close to the 3' end can still be targeted in the second half and, if necessary, a novel intron insertion site can be generated by introducing silent mutations. Of course, through genetic methods any site can be targeted with a little more effort than the analyzed sites found with these criterions that provide easy access for SCON insertion or intron replacement.

### Recapitulation

In summary, SCON-mediated cKO approach renders the complicated generation of conditional allele into a simple CRISPR-mediated short sequence knock-in of 189 bp intronic sequence in the optimized SCON variant with one-step zygote injection. The intron is neutral on expression levels before Cre-mediated inactivation. This novel strategy opens an exciting possibility of applying the same strategy to other vertebrate models ranging from fish to non-human primates. Moreover, the LoxP sequences can also be replaced by other recombination sites (e.g. FRT) for rapid generation of FRT- or other recombinase-based conditional alleles that have not been widely utilized yet. Lastly, the dispensable region between the two LoxP sites serve as a harboring space for an addition of other genetic elements. The SCON strategy can be a new foundation of cKO approach in biomedical and industrial research that is well suited for animal welfare.

## Claims

1. A genetic element comprising:
a splice donor site, a first recombinase recognition site, a splice branch point, a second recombinase recognition site, a splice acceptor site, wherein the splice branch point is at a distance of 10 to 56 nucleotides in length from the splice acceptor site, or a reverse complementary sequence thereto.

2. The genetic element of claim 1, wherein the splice branch point is at a distance of 0 to 11 nucleotides in length from the second recombinase recognition site.

3. The genetic element of claim 1 or 2, wherein the splice donor site is at a distance of 80 to 5000 nucleotides in length from the splice acceptor site.

4. The genetic element of any one of claims 1 to 3, wherein the sequence of 10 nucleotides directly 5' adjacent to the splice acceptor site contains at least 8 pyrimidine nucleotides, preferably of which at least 3 nucleotides are C and/or preferably at least 3 nucleotides are T.

5. The genetic element of any one of claims 1 to 4, wherein the splice donor site comprises the nucleic acid sequence GTPuAG, with Pu being a purine base, the splice branch point comprises the nucleic acid sequence CTPuAPy, with Pu being a purine base and Py being a pyrimidine base, the splice acceptor site comprises the sequence AG, or combinations thereof.

6. The genetic element of any one of claims 1 to 5, wherein the first and second recombinase recognition sites are selected from a tyrosine recombinase site, preferably a loxP or FRT site, especially preferred a loxP site, even more preferred a loxP site comprising the nucleic acid sequence ATAACTTCGTATAAGGTATCCTATACGAAGTTAT (SEQ ID NO: 17); a lox 66 site or a lox 71 site, a FRP site, a FRT suite, especially preferred a FRT site comprising the nucleic acid sequence GAAGTTCCTATTCTCTAGAAAGTATAGGAACTTC (SEQ ID NO: 18).

7. The genetic element of any one of claims 1 to 6, comprising two or more splice branch points, preferably wherein two splice branch points are at a distance of 1 to 10 nucleotides in length to each other.

8. A genetic vector, preferably an expression or integration vector, a single strand DNA oligo template, a double strand DNA template, a transposon, or a viral vector, comprising the genetic element of any one of claims 1 to 7.

9. A method of providing a cell with a conditionally deactivat-able gene, comprising providing a cell, introducing the genetic element of any one of claims 1 to 7 into an exon of a gene in a cell, or introducing a gene with the genetic element of any one of claims 1 to 7 into a cell, preferably with a vector according to claim 8.

10. The method of claim 9, wherein the introduction into an exon of a gene comprises CRISPR-Cas, preferably CRISPR-Cas, especially preferred CRISPR-Cas9, mediated insertion.

11. A cell comprising a gene with two or more exons and at least one intron, wherein the intron comprises the genetic element of any one of claims 1 to 7 and wherein the intron is located between two exons.

12. A non-human animal comprising one or more cells of claim 11.

13. A method of inactivating expression of a functional gene in a cell or non-human animal, comprising providing a cell of claim 11 or a non-human animal of claim 12 and activating recombination at the recombinase recognition sites in the cell of claim 11 or in a cell in the non-human animal of claim 12.

14. A method of investigating the function of a gene, comprising inactivating a functional gene according to the method of claim 13 and comparing the inactivated gene's effect in the cell or non-human animal to a cell or non-human animal without inactivation of the gene or to a cell or non-human animal without the genetic element of any one of claims 1 to 7.

15. A kit suitable for integrating an intron into a target gene comprising a genetic element of any one of claims 1 to 7 and a Cas encoding nucleic acid, preferably further comprising a CRISPR-Cas guide nucleic acid targeting an exon in the target gene.

16. a method of introducing an intron sequence into an exon or between two exons of a gene, comprising the steps of selecting the exon or one of the two exons, respectively, which is positioned within the first 50% base pairs (bp) of a protein coding-sequence of the gene; and wherein the intron is inserted into an intron insertion site containing either a stringent splice junction consensus sequence or a flexible splice junction consensus sequence; and wherein after the introduction of the intron the intron is separating exons on the intron's 5' and 3' sides with the exons being each at least 60 bp in length, preferably wherein the intron sequence is a genetic element of any one of claims 1 to 7.
